(19) Europäisches Patentamt / European Patent Office / Office européen des brevets

(11) **EP 4 101 933 A1**

(12) **EUROPEAN PATENT APPLICATION**
published in accordance with Art. 153(4) EPC

(43) Date of publication:
**14.12.2022 Bulletin 2022/50**

(21) Application number: **21751230.0**

(22) Date of filing: **06.02.2021**

(51) International Patent Classification (IPC):
*C12Q 1/6809* (2018.01)   *C12Q 1/6869* (2018.01)

(52) Cooperative Patent Classification (CPC):
**C12Q 1/6809; C12Q 1/6869; Y02A 90/10**

(86) International application number:
**PCT/JP2021/004470**

(87) International publication number:
**WO 2021/157739 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.02.2020 JP 2020018989**

(71) Applicant: **Karydo Therapeutix, Inc.**
**Tokyo 102-0082 (JP)**

(72) Inventor: **SATO, Narutoku**
**Soraku-gun, Kyoto 619-0288 (JP)**

(74) Representative: **Vos, Derk**
**Maiwald GmbH**
**Elisenhof**
**Elisenstraße 3**
**80335 München (DE)**

(54) **CORRECTION METHOD FOR SINGLE-CELL RNA-SEQ ANALYSIS COUNT DATA SET, ANALYSIS METHOD FOR SINGLE-CELL RNA-SEQ, ANALYSIS METHOD FOR CELL TYPE RATIOS, AND DEVICES AND COMPUTER PROGRAMS FOR EXECUTING SAID METHODS**

(57)    Disclosed in the description is a method for correcting a count data set for single-cell RNA-Seq analysis, including weighting a count data set for single-cell RNA-Seq analysis obtained from cells to be analyzed or predicted for the cells to be analyzed based on the total RNA content of each cell type corresponding to the cells to be analyzed.

**Description**

Technical Field

This description discloses a method for correcting a count data set for single-cell RNA-Seq analysis, a method for analyzing single-cell RNA-Seq, a method for analyzing composition ratios of cell types, and devices and computer programs for performing these methods.

Background Art

A human organ is composed of about $1 \times 10^8$ to $3 \times 10^{12}$ cells. A change in cellular composition and/or cellular phenotype of an organ is closely interrelated with its dysfunction, remodeling and regeneration. Each individual organ is a mixed population of cells. Thus, in order to capture a change in cellular composition and/or cellular phenotype of an organ, single-cell RNA-Seq (or scRNA-Seq) analyzes a comprehensive gene expression profile for the cell population of each organ, and breaks down the analysis data into the expression levels of single cells to derive information about changes in single cells (Non-Patent Document 1 to Non-Patent Document 5). Thus, scRNA-Seq is said to be a powerful method for generating detailed molecular cell atlases of normal and abnormal organs.

However, scRNA-Seq has its limitations. First, for use in scRNA-Seq, individual cells must be recovered from the tissue collected from an organ using a digestive enzyme or by physical disruption. The premise of this is that such cells cannot be recovered unless the tissue is fresh. In other words, tissues generally collected by surgery or the like are often cryopreserved for several months to several years, and such preserved tissues cannot be used for scRNA-Seq. Even if a rare disease is found in a histopathological diagnosis after surgery, it is difficult to newly obtain such rare pathological samples, and the samples that can be used for RNA expression analyses have been usually cryopreserved. Also, tissues are usually collected from humans by biopsy, and the problem is that the volume of sample is small. Even if the entire organ can be collected by autopsy or the like, it would be impractical, if not impossible, to isolate individual cells from the entire organ for the purpose of scRNA-Seq in the case of a large organ such as heart or brain.

In addition, the problem in many cases is that it is necessary to analyze drug-induced effects and/or pathological conditions in multiple different organs of the same subject in a study of drug effects and/or etiology, but, in the case of humans, it is difficult to collect multiple types of organs for analysis from one subject.

Further, scRNA-Seq has a problem of artifacts related to the experimental method in gene expression. As such an example, it has been reported that abnormal gene expression is induced in cells during the step of isolating cells.

For the purpose of solving the above problems, computerized whole-organ RNA database deconvolution (computational deconvolution of whole-organ RNA datasets has been proposed. Whole-organ RNA database deconvolution is a method in which RNAs are extracted from the collected test tissue without cell isolation for each cell type to obtain information about expressed RNA-sequences by RNA-Seq, and then the RNA expression level is estimated for each cell type based on the proportions of cell types contained in the test tissue calculated by a computer. This method allows an RNA expression analysis not only for fresh tissues but also for cryopreserved tissues. Also, this method allows simultaneous purification of RNAs from multiple organs.

Several computer analysis methods for deconvolution of whole-organ RNA-Seq data have been proposed so far (Non-Patent Documents 6 to 19). These methods use almost the entire RNA-Seq data of the corresponding organ to calculate the composition of cell types in the organ to be analyzed.

Recently, methods called MUlti-Subject Single Cell deconvolution (MuSiC) (Non-Patent Document 17), Dampened Weighted Least Squares (DWLS) (Non-Patent Document 18), and Complete Deconvolution for Sequencing data (CDSeq) (Non-Patent Document 19) were reported. It is said that these three methods are superior to the previously reported methods described in Non-Patent Documents 6 to 16.

Citation List

Non-Patent Document

Non-Patent Document 1: Deng, Q., Ramskold, D., Reinius, B. & Sandberg, R. Science 343, 193-196, doi:10.1126/science.1245316 (2014).
Non-Patent Document 2: Han, X. et al. Mapping the Mouse Cell Atlas by Microwell-Seq. Cell 172, 1091-1107 e1017, doi:10.1016/j.cell.2018.02.001 (2018).
Non-Patent Document 3: Regev, A. et al. Science Forum: The Human Cell Atlas. Elife 6, doi:10.7554/eLife.27041 (2017).

Non-Patent Document 4: Sandberg, R. Nature methods 11, 22-24, doi:10.1038/nmeth.2764 (2014).

Non-Patent Document 5: Tabula Muris, C. et al., Nature 562, 367-372, doi:10.1038/s41586-018-0590-4 (2018).

Non-Patent Document 6: Abbas, A. R. et al., PloS one 4, e6098, doi:10.1371/journal.pone.0006098 (2009).

Non-Patent Document 7: Avila Cobos, F. et al., Bioinformatics 34, 1969-1979, doi:10.1093/bioinformatics/bty019 (2018).

Non-Patent Document 8: Gaujoux, R. & Seoighe, C.,Infect Genet Evol 12, 913-921, doi:10.1016/j.meegid.2011.08.014 (2012).

Non-Patent Document 9: Gong, T. et al. PloS one 6, e27156, doi:10.1371/journal.pone.0027156 (2011).

Non-Patent Document 10: Gong, T. & Szustakowski, J. D.,Bioinformatics 29, 1083-1085, doi:10.1093/bioinformatics/btt090 (2013).

Non-Patent Document 11: Li, B. et al., Genome biology 17, 174, doi:10.1186/s13059-016-1028-7 (2016).

Non-Patent Document 12: Newman, A. M. et al.,Nature methods 12, 453-457, doi:10.1038/nmeth.3337 (2015).

Non-Patent Document 13: Repsilber, D. et al., BMC bioinformatics 11, 27, doi:10.1186/1471-2105-11-27 (2010).

Non-Patent Document 14: Shen-Orr, S. S. & Gaujoux, R., Curr Opin Immunol 25, 571-578, doi:10.1016/j.coi.2013.09.015 (2013).

Non-Patent Document 15: Wang, N. et al. , Bioinformatics 31, 137-139, doi:10.1093/bioinformatics/btu607 (2015).

Non-Patent Document 16: Zhong, Y. et al., BMC bioinformatics 14, 89, doi:10.1186/1471-2105-14-89 (2013).

Non-Patent Document 17: Tsoucas, D. et al., Nat Commun 10, 2975, doi:10.1038/s41467-019-10802-z (2019).

Non-Patent Document 18: Wang, X. et al., Nat Commun 10, 380, doi:10.1038/s41467-018-08023-x (2019).

Non-Patent Document 19: Kang, K. et al., PLoS computational biology 15, e1007510, doi:10.1371/journal.pcbi. 1007510 (2019).

Summary of Invention

Technical Problem

However, the methods described in Non-Patent Documents 17 to 19 have been merely validated for their usefulness in RNA-Seq data derived from synthesis data sets, cultured cells, mixtures of several tissues, and/or one to four real organs. In other words, the applicability to a wider variety of real organs has not been explored. The present inventor evaluated the performance of the MuSiC method (Non-Patent Document 17) and the DWLS method (Non-Patent Document 19). These are the two newest methods that perform deconvolution on one to four real organs and have been compared to and shown to be superior to other previous methods. However, as shown in the verification of the effects described later, the ratio of cell types calculated by a computer in the MuSiC or DWLS method deviated from those experimentally estimated by actual scRNA-Seq studies, and the degree of deviation varied. In particular, the deviations were pronounced for skeletal muscle and heart.

Therefore, in order to eliminate such deviation, an object of the present invention is to provide an RNA-Seq data deconvolution method for estimating the proportions of respective cell types that are closer to the proportions of respective cells in real tissues. Another object is to provide an RNA-Seq data deconvolution method that is applicable to a wider variety of tissues.

Solution to Problem

A certain embodiment of the present invention relates to a method for correcting a count data set for single-cell RNA-Seq analysis, including: weighting a count data set for single-cell RNA-Seq analysis obtained from cells to be analyzed or predicted for the cells to be analyzed based on the total RNA content of each cell type corresponding to the cells to be analyzed.

Preferably, the weighting is performed based on the expression of a signature gene set that characterizes each cell type, and the signature gene set includes a predetermined number of genes.

A certain embodiment of the present invention relates to a method for analyzing single-cell RNA-Seq, including: weighting a count data set for single-cell RNA-Seq analysis obtained from cells to be analyzed or predicted for the cells to be analyzed based on the total RNA content of each cell type corresponding to the cells to be analyzed, and analyzing an RNA expression pattern in each cell type composing an organ to be analyzed containing the cells to be analyzed based on the weighted count data set for single-cell RNA-Seq analysis.

A certain embodiment of the present invention relates to a method for analyzing the composition ratios of cell types composing an organ to be analyzed, including: weighting a count data set for single-cell RNA-Seq analysis obtained from cells to be analyzed or predicted for the cells to be analyzed based on the total RNA content of each cell type corresponding to the cells to be analyzed, and analyzing the composition ratios of cell types composing an organ to be analyzed containing the cells to be analyzed based on the weighted count data set for single-cell RNA-Seq analysis.

A certain embodiment of the present invention relates to a device (10) for correcting a count data set for single-cell RNA-Seq analysis. The correcting device (10) includes a control part (101). The control part (101) weights a count data set for single-cell RNA-Seq analysis acquired from cells to be analyzed based on the total RNA content of each cell type corresponding to the cells to be analyzed.

A certain embodiment of the present invention relates to a device for analyzing single-cell RNA-Seq. The analyzing device (20) includes a control part (201). The control part (201) weights a count data set for single-cell RNA-Seq analysis obtained from cells to be analyzed or predicted for the cells to be analyzed based on the total RNA content of each cell type corresponding to the cells to be analyzed, and analyzes an RNA expression pattern in each cell type composing an organ to be analyzed containing the cells to be analyzed based on the weighted count data set for single-cell RNA-Seq analysis.

A certain embodiment of the present invention relates to a device for analyzing the composition ratios of cell types composing an organ to be analyzed. The analyzing device (20) includes a control part (201). The control part (201) weights a count data set for single-cell RNA-Seq analysis obtained from cells to be analyzed or predicted for the cells to be analyzed based on the total RNA content of each cell type corresponding to the cells to be analyzed, and analyzes the composition ratios of cell types composing an organ to be analyzed containing the cells to be analyzed based on the weighted count data set for single-cell RNA-Seq analysis.

A certain embodiment of the present invention relates to a program for correcting a count data set for single-cell RNA-Seq analysis, executable by a computer to cause the computer to execute processing including a step of weighting a count data set for single-cell RNA-Seq analysis obtained from cells to be analyzed or predicted for the cells to be analyzed based on the total RNA content of each cell type corresponding to the cells to be analyzed.

A certain embodiment of the present invention relates to a program for analyzing single-cell RNA-Seq, executable by a computer to cause the computer to execute processing including steps of weighting a count data set for single-cell RNA-Seq analysis obtained from cells to be analyzed or predicted for the cells to be analyzed based on the total RNA content of each cell type corresponding to the cells to be analyzed, and analyzing an RNA expression pattern in each cell type composing an organ to be analyzed containing the cells to be analyzed based on the weighted count data set for single-cell RNA-Seq analysis.

A certain embodiment of the present invention relates to a program for analyzing the composition ratios of cell types composing an organ to be analyzed, executable by a computer to cause the computer to execute processing including the steps of weighting a count data set for single-cell RNA-Seq analysis obtained from cells to be analyzed or predicted for the cells to be analyzed based on the total RNA content of each cell type corresponding to the cells to be analyzed, and analyzing the composition ratios of cell types composing an organ to be analyzed containing the cells to be analyzed based on the weighted count data set for single-cell RNA-Seq analysis.

Advantageous Effects of Invention

The present invention makes it possible to estimate the proportions of respective cell types closer to the proportions of respective cells in real tissues from an RNA sequence database. Also, according to the present invention, it is possible to estimate the proportions of respective cell types in wider variety of tissues.

Brief Description of Drawings

FIG. 1 shows an example of a hardware configuration of a correcting device 10.
FIG. 2 shows the flow of processing by a correction program 1042.
FIG. 3 shows an example of a hardware configuration of an analyzing device 20.
FIG. 4 shows the flow of processing by an analysis program 2042.
FIG. 5 shows the composition ratios of reference cell types of respective cell types present in respective organs (aorta, brain, fat, heart, kidney, large intestine, liver and lung), the composition ratios of cell types predicted by the MuSiC method, and the composition ratios of cell types predicted by the DWLS method.
FIG. 6 shows the composition ratios of reference cell types of respective cell types present in respective organs (bone marrow, pancreas, skin, skeletal muscle, spleen and thymus), the composition ratios of cell types predicted by the MuSiC method, and the composition ratios of cell types predicted by the DWLS method.
FIG. 7 shows comparison between an estimated whole-organ RNA-Seq data set obtained from the composition ratios of reference cell types and real scRNA-Seq data of respective organs, and a real whole-organ RNA-Seq data set.
FIG. 8 shows weight coefficients of respective cell types present in respective organs and their distribution ranges.
FIG. 9 shows comparison between an estimated whole-organ RNA-Seq data set estimated using cell type-specific weight coefficients obtained in the present invention and real whole-organ RNA-Seq data set.

FIG. 10 shows an overview of a whole-organ RNA-Seq data deconvolution method according to the present invention. In the drawing, w represents a weight, m represents the RNA count of each gene, and n represents the ratio of each cell type.

FIG. 11 shows the composition ratios of reference cell types of respective cell types present in respective organs (aorta, fat, heart, kidney, liver, lung, large intestine, bone marrow, skeletal muscle and spleen), the composition ratios of respective cells estimated according to the present invention, the composition ratios of cell types predicted by the MuSiC method, and the composition ratios of cell types predicted by the DWLS method.

FIG. 12 shows mean square errors (MSEs) of the composition ratios of respective cells estimated according to the present invention, the composition ratios of cell types predicted by the MuSiC method and the composition ratios of cell types predicted by the DWLS method relative to the composition ratios of reference cell types.

FIG. 13 shows comparison between estimated transcript counts in aorta, fat, heart, kidney, liver, lung, large intestine, bone marrow, skeletal muscle and spleen, and gene expressions of respective cell types in real organs.

FIG. 14 shows results of t-Distributed Stochastic Neighbor Embedding (t-SNE) analysis on estimated scRNA-Seq count data.

FIG. 15 shows results of estimation of the composition ratios of cell types in heart and gene expression profiles in respective cell types performed using mouse models with myocardial infarction (MI) according to the present invention. FIG. 15a shows the rates of change in estimated composition ratios of cell types relative to Sham. FIG. 15b shows results of variation analysis of estimated gene expression profiles.

FIG. 16 shows results of deconvolution of a human whole-organ RNA-Seq data set performed using weight coefficients calculated using data of mice and estimated scRNA-Seq count data. FIG. 16a shows the composition ratios of cell types estimated for human heart and kidney. FIG. 16b shows results of t-Distributed Stochastic Neighbor Embedding (t-SNE) analysis of gene expression profiles estimated for human heart and kidney.

Description of Embodiments

1. Correction method, analysis method, and method for analyzing composition ratios of cell types composing organ to be analyzed

A certain embodiment of the present invention relates to a method, device and program for correcting a count data set for single-cell RNA-Seq analysis.

1-1. Method for correcting count data set for single-cell RNA-Seq analysis

The method for correcting a count data set for single-cell RNA-Seq (scRNA-Seq) analysis (which is hereinafter also referred to simply as "correction method") includes weighting a count data set for single-cell RNA-Seq analysis obtained from the cells to be analyzed or predicted for the cells to be analyzed based on the total RNA content of each cell type.

1-1-1. RNA-Seq analysis

In this description, RNAs are not limited as long as they are RNAs that can be analyzed by RNA-Seq analysis. The RNAs may include mRNAs, untranslated RNAs, microRNA, and so on.

The RNAs are not limited as long as they are present in organisms. The organisms are not limited as long as they are multicellular organisms having organs. The organisms may be plants or animals, but is preferably animals. Preferably, the animals are mammals such as humans, mice, rats, dogs, cats, rabbits, cows, horses, goats, sheep and pigs, or birds such as chickens. The animals are more preferably mammals such as humans, mice, dogs, cats, cows, horses and pigs, still more preferably humans, mice, dogs, cats or the like, much more preferably humans or mice, and most preferably humans. Also, the organisms include both diseased and non-diseased organisms.

The cells to be analyzed are not limited as long as they are present in organs of the organisms. Preferably, the organs are organs with known cellular composition therein.

An organ means an assembly of several tissues present in an organism and having a certain independent form and a specific function. For example, when the organisms are mammals, the term "organ" may include circulatory system organs (heart, artery, vein, lymph duct, etc.), respiratory system organs (nasal cavity, paranasal sinus, larynx, trachea, bronchi, lung, etc.), gastrointestinal system organs (lip, cheek, palate, tooth, gum, tongue, salivary gland, pharynx, esophagus, stomach, duodenum, jejunum, ileum, cecum, appendix, ascending colon, transverse colon, sigmoid colon, rectum, anus, liver, gallbladder, bile duct, biliary tract, pancreas, pancreatic duct, etc.), urinary system organs (urethra, bladder, ureter, kidney), nervous system organs (cerebrum, cerebellum, mesencephalon, brain stem, spinal cord, peripheral nerve, autonomic nerve, etc.), female reproductive system organs (ovary, oviduct, uterus, vagina, etc.), breast, male reproductive system organs (penis, prostate, testicle, epididymis, vas deferens), endocrine system organs (hypothalamus, pituitary gland, pineal body, thyroid gland, parathyroid gland, adrenal gland, etc.), integumentary system organs (skin, hair, nail, etc.), hematopoietic system organs (blood, bone marrow, spleen, etc.), immune system organs (lymph node, tonsil, thymus, etc.), bone and soft tissue organs (bone, cartilage, skeletal muscle, connective tissue, ligament, tendon, diaphragm, peritoneum, pleura, adipose tissue (brown adipose, white adipose), etc.), sensory system organs (eyeball, palpebra, lacrimal gland, external ear, middle ear, inner ear, cochlea, etc.), and so on. In the present invention, the tissue of interest is preferably that of heart, cerebrum, lung, kidney, adipose tissue, liver, skeletal muscle, testicle, spleen, thymus, bone marrow, pancreas, or skin (including epidermis above the subcutaneous tissue, papillary layer and plexiform layer). Preferred organs are aorta, brain, fat, heart, kidney, large intestine, liver, lung, bone marrow, pancreas, skin, skeletal muscle, spleen and thymus.

RNA-Seq analysis is a so-called transcriptome analysis, which is a method for analyzing the expressed genes or the number of counts (also called the number of read counts) thereof by comprehensively acquiring reads including sequence information from RNAs present in a sample of interest and mapping the reads on a reference sequence. The number of counts corresponds to the gene expression level. The count data for RNA-Seq analysis may include the gene names of expressed genes and/or registration numbers thereof in a gene database, and the numbers of counts of reads of respective genes.

RNA-Seq analysis can be performed using a DNA sequencer called next generation sequencer or third generation sequencer. Examples of next generation sequencers include MiSeq9 (trademark), HiSeq (trademark), NextSeq (trademark) and MiSeq (trademark) available from Illumina, Inc. (San Diego, CA); Ion Proton (trademark) and Ion PGM (trademark) available from Thermo Fisher Scientific (Waltham, MA); GS FLX+ (trademark) and GS Junior (trademark) available from Roche (Basel, Switzerland), and so on. Examples of third generation sequencers include PacBio Sequel (tradename) and so on.

A count data set for scRNA-Seq analysis is a set of count data generated based on gene expressions predicted by expression analysis of genes expressed in individual cells of an organism and/or a computer analysis method. For example, a count data set for scRNA-Seq analysis may be count data acquired from real individual cells by RNA-Seq analysis. Also, a count data set for scRNA-Seq analysis may be a count data set predicted by performing, for example, deconvolution on count data acquired from a whole organ by RNA-Seq analysis based on reference cell composition ratios by a computer analysis method according to the method described in Non-Patent Documents 6 to 19. As a method for predicting a count data set for scRNA-Seq analysis, a method called Complete Deconvolution for Sequencing data (CDSeq) (Non-Patent Document 19), for example, is preferred.

1-1-2. Calculation of weight coefficients based on RNA contents in different cell types present in each organ

A method for calculating weight coefficients for weighting a count data set for single-cell RNA-Seq analysis obtained from the cells to be analyzed or predicted for the cells to be analyzed based on the total RNA content of each cell type is described.

First, prior to weighting, it is necessary to acquire information about the cell types of which each organ is composed. The cellular composition of each organ can be acquired from scRNA-Seq data described in Non-Patent Document 5 or Non-Patent Document 2, or from a database registered in NIH or the like. These compositions of cell types are information obtained by actually analyzing the compositions of cell types of the tissues of each organ. Such a cellular composition of each organ is also referred to as "reference cell types." The reference cell types include a count data set for scRNA-Seq about genes that are usually expressed in each cell type. Also, the reference cell types include the composition ratios of reference cell types in each organ (also referred to as "references"), which are linked with labels indicating the names or abbreviated names of respective cell types.

In the calculation of weight coefficients, it is preferred to use the composition of cell types in each organ described in Non-Patent Document 5 as reference cell types and their composition ratios for aorta, fat, kidney, large intestine, liver, lung, bone marrow, pancreas, skin, spleen and thymus.

Also, for skeletal muscle, it is preferred to use the ratios of cell types described in Non-Patent Document 2 as reference cell types and their composition ratios.

For heart, it is preferred to correct the composition ratios of reference cell types described in Non-Patent Document 5 in connection with the separation analysis between cardiac muscle cells and non-muscle cells and use them as the composition ratios of reference cell types. Specifically, the composition ratio (3.1%) of cardiac muscle cells adopted in Non-Patent Document 5 is extremely low compared to the rates (30% to 40%) that have been generally consented based on various previous studies in the field of histological anatomy. Thus, in this embodiment, it is preferred to set the composition ratio of cardiac muscle cells to 30% and to obtain the composition ratios of reference cell types by dividing the remaining 70% by the composition ratios of non-muscle cell types.

For brain, it is preferred to determine the reference cell types and their composition ratios based on a report in NIH (http://www.nervenet.org/papers/BrainRev99.html#Numbers). As labels indicating respective cell types in brains, corresponding cell type labels of scRNA-Seq data described in Non-Patent Document 5 were used. First, the cell type classes in brains are classified into four classes; "neurons," "glial cells," "endothelial cells," and "others", and the ratio of respective classes is set to 75:23:7:4. This ratio is determined according to the estimated ratio in brains of mice (http://www.nervenet.org/papers/BrainRev99.html#Numbers). Next, according to Non-Patent Document 5, the classes of "neurons," "glial cells" and "others" are classified into more detailed cell type classes. Specifically, the class of "neurons" is further classified into "nerve cells-excitable neurons and several neural stem cells" and "nerve cells-inhibitory neurons." The class of "others" is classified into "brain pericytes-NA" and "oligodendrocyte precursor cells-NA." The class of "glial cells" is classified based on the following three premises. i) The class of "glial cells" is classified into four cell types according to Non-Patent Document 5; "microglial cells-NA," "astrocytes-NA," "Bergmann glial cells-NA" and "oligodendrocytes-NA." ii) The composition ratios of these four glial cell types follow the description in Non-Patent Document 5. iii) Because "microglial cells" are reported to account for 10 to 15% of cells in the whole brain, the ratio of "microglial cells-NA" in the whole brain is set to 0.1. Based on these premises, the rates of respective brain cell types are set to as follows; "macrophages-NA" (approximately 0.2%), "microglial cells-NA" (10.0%), "astrocytes-NA"(approximately 2.2%), "Bergmann glial cells-NA"(approximately 2.1%), "brain pericytes-NA" (approximately 1.5%), "endothelial cells-NA" (approximately 6.4%), "nerve cells-excitable neuron and several neural stem cells" (approximately 47.5%), "nerve cells-inhibitory neurons "(approximately 21.3%), "oligodendrocytes-NA" (approximately 8.7%), and "oligodendrocyte precursor cells-NA" (approximately 1.9%). These can be used as the reference cell types of brain and their composition ratios.

The reference cell types used in this description, and the composition ratios of the cell types are shown in the list of composition ratios of reference cell types at the end of this document.

Further, for the calculation of weight coefficients, in addition to the composition ratios of reference cell types as described above, the gene expression in each cell type, in other words, count data for scRNA-Seq analysis in each cell type is required. However, there are generally 20000 to 30000 genes that are subject to scRNA-Seq analysis.

Although the count data of all these genes may be used, it is more efficient to select genes that can characterize each cell type (signature genes) and use the count data of the gene set to calculate weight coefficients. Such a signature gene set that characterizes each cell type can be calculated by the following method, for example.

First, before selecting signature genes, it is preferred to delete the counts of spike-in genes with an ERCC label to be attached thereto and the counts derived from the three genes Rn45s, Akap5 and Lrrc17, which significantly affect the total count but are reported as non-mRNA artifacts, from the count data for scRNA-Seq analysis. Also, it is preferred to normalize the RNA count derived from each gene by converting it such that the total count of each cell in the scRNA-Seq data set is 100, $10^3$, $10^4$, $10^5$, $10^6$ or the like.

For the selection of signature genes, a classifier generated by training an artificial intelligence such as random forest, for example, can be used. The composition ratios of reference cell types in each organ and a count data set for scRNA-Seq analysis reported for each reference cell type are used to train an artificial intelligence to generate a classifier. For example, when random forest is used as an artificial intelligence, important feature amounts of the classifier were extracted as signature gene names of each cell type, and a "Mean Decrease Gini" value was used as an importance index of each gene to extract genes with a high "Mean Decrease Gini" value as signature genes. About 100 to 2000 genes can be extracted in descending order of the "Mean Decrease Gini" value as signature genes and used as a signature gene set.

Next, for each cell type present in each organ, a weight coefficient for correcting the count data set for scRNA-Seq analysis with the RNA content is calculated.

For the calculation of the weight coefficient, count data for scRNA-Seq analysis of a signature gene set in each cell type of reference cell types (which is also referred to as "signature gene scRNA-Seq data"), and count data obtained by RNA-Seq analysis of the total RNAs contained in the whole of each organ (which is also referred to as "whole-organ RNA-Seq data") can be used for each organ. The signature gene scRNA-Seq count data and the whole-organ RNA-Seq count data are both normalized before use.

As the whole-organ RNA-Seq data, a disclosed count data set for RNA-Seq analysis can be used. The whole-organ RNA-Seq data of mice can be acquired from "i-organs.atr.jp." The human whole-organ RNA-Seq data can be acquired from "The Human Protein Atlas" (https://www.proteinatlas.org/; heart (ERR315328) and kidney (ERR315494)).

The weight coefficients can be calculated according to the following method.

[Math. 1]

$$\mathbf{y} \in \mathbb{R}^n, \, w_j > 0, \text{ and } \mathbf{x}_j \in \mathbb{R}^n$$

represent a vector of normalized counts for whole-organ RNA-Seq analysis, a weight coefficient for each cell j to be analyzed, and a matrix of normalized counts for scRNA-Seq analysis, respectively. Here, n represents the number of genes of signature genes of each organ. In addition, a combination $C^m$ of cells to be analyzed is randomly selected under the restriction that the composition ratios of reference cell types are kept within a total set size m. Also, when a count data set for scRNA-Seq predicted for the cells to be analyzed is used, a matrix of count data for scRNA-Seq predicted for the cells to be analyzed is used instead of the matrix of a normalized count for scRNA-Seq analysis.

Next, the following formula (1) is described.

[Math. 2]

$$my = \sum_j w_j x_j \ (j \in C^m) \tag{1}$$

In formula (1), m represents a multiplying factor, which is determined depending on n. m is set to a value smaller than n in each of the following calculations. Here, $w_j$ is calculated by solving a quadratic programming problem according to the following formula (2) under the restriction that the resulting value is 0.01 or greater.

[Math. 3]

$$\widetilde{w}_j = \mathrm{argmin}_{w_j} \sum_i^S \left| m y_i - \sum_j w_j x_j \right|^2 \ s.t. \ w_j \geq 0.01. \tag{2}$$

In formula (2), S represents the number of count data sets for RNA-Seq of each gene targeting the whole organ. For example, when corresponding count data sets for whole-organ RNA-Seq acquired from different two individuals are used, S is 2. This quadratic programming problem can be solved using a "quadprog" package in R. Both the steps of randomly selecting combinations of cells to be analyzed and calculating [Math. 4] $\widetilde{w}_j$ are recursively done for all the selected cells to be analyzed until the number of $w_j$ reaches 100 or more.

1-1-3. Correction of count data set for scRNA-Seq analysis

Using the calculated weight coefficients, weighting is performed on a count data set for scRNA-Seq obtained from the cells to be analyzed or predicted for the cells to be analyzed based on the total RNA content of each cell type corresponding to the cells to be analyzed.

For weighting, on the premise that the distribution of weight coefficients follows a Gaussian distribution, the mean and variance of weighted counts of genes of respective cells to be analyzed are calculated according to the following formula (3).

[Math. 5]

$$\tilde{x}_j \sim \mathcal{N}\left( \mu_{w_j} x_j, \sigma^2_{w_j} \mathrm{diag}(x_j \odot x_j) \right) \tag{3}$$

In formula (3),

[Math. 6] $\tilde{x}_j$, $\mu_{wj}$, and $\sigma^2_{wj}$

represent a weighted count vector of genes of the cells j to be analyzed, the mean of weight coefficients for cells j to be analyzed, and the variance of weight coefficients for cells j to be analyzed, respectively.

[Math. 7] An operator $\odot$

represents an element-wise product between two vectors.

Based on the calculated mean and variance of weight coefficients of respective cells to be analyzed, the mean and variance of weight coefficients in the corresponding cell types are calculated according to the following formula (4) on the premise that the mean and variance of weight coefficients in the corresponding cell types follow a Gaussian distribution.

[Math. 8]

$$\bar{x}_k \sim \mathcal{N}\left(\frac{1}{N_k}\sum_j \mu_{w_j} x_j \,,\, \frac{1}{N_k}\sum_j \sigma^2_{w_j} \mathrm{diag}(x_j \odot x_j)\right) (j \in C^k). \qquad (4)$$

In formula (4), k, $C^k$ and $N_k$ represent a cell type, the group of cells to be analyzed labeled to the cell type k, and the numbers of the cells to be analyzed in $C^k$, respectively.

The mean, variance and quartiles of the weight coefficients for respective cell types present in respective organs calculated according to the above formula (2) are shown in the weight coefficient list described later.

The count data set for scRNA-Seq analysis weighted by this method is also referred to as "estimated scRAN-Seq count data set."

2. Analysis of composition ratios of cell types in each organ and analysis of total RNA expression patterns in the cell types

Using the weight coefficients calculated in section 1-1-2. above, the composition ratios of cell types composing an organ to be analyzed can be analyzed. The analysis of composition ratios of cell types composing an organ to be analyzed includes calculating the composition ratios of cell types composing an organ to be analyzed containing cells to be analyzed based on a count data set for scRNA-Seq analysis weighted in Section 1-1-3. above. In other words, the composition ratios of cell types acquired by this method are estimated composition ratios.

Also, using the weight coefficients calculated in Section 1-1-2. above, the total RNA expression patterns in cell types composing an organ to be analyzed can be analyzed. The analysis of total RNA expression patterns is to acquire estimated count data for scRNA-Seq analysis. Here, the term "total RNA" is intended to include RNAs expressed from a signature gene set and other genes.

For example, the analysis of the composition ratios of cell types and total RNA expression pattern in each cell type can be calculated simultaneously by designing an algorithm based on the Bayes' theorem.

The calculation can be done according to the following formula (5).
[Math. 9]

$$y = Xr \qquad (5)$$

In formula (5),

[Math. 10]
$$y, X = (\bar{x}_1, \dots, \bar{x}_k, \dots, \bar{x}_K)$$

, and r

represent a whole-organ RNA-Seq data vector, a matrix including estimated scRNA-Seq data, which are weighted counts calculated using weight coefficients for respective cell types according to the above formula (4), in its columns, and a coefficient vector corresponding to the composition ratios of cell types, respectively. The counts weighted with the weight coefficients for respective cell types calculated according to the above formula (4) are initial values, and updated to new values by the calculation of formula (7) described later. For the calculation of X and r, the Bayes' theorem is used. In order to apply the Bayes' theorem,

[Math. 11] noise $\mathcal{N}(0, \beta^{-1} I)$

is added to formula (5), and a probabilistic model shown in the following formula (6) is adopted.
[Math. 12]

$$P(y|X, r) = \mathcal{N}(y|Xr, \beta^{-1} I) \qquad (6)$$

In formula (6), $\beta$ represents a hyperparameter for controlling the degree of variation of the distribution in estimating the gene expression pattern of each cell type. According to the Bayes' theorem, the posterior distributions of X and r are obtained as the following formula (7).
[Math. 13]

$$P(\mathbf{X}, \mathbf{r}|\mathbf{y}) \propto P(\mathbf{y}|\mathbf{X}, \mathbf{r})P(\mathbf{X})P(\mathbf{r}) \tag{7}$$

In formula (7), P(X) and P(r) represent prior distributions of X and r, respectively. P(X) and P(r) are given as the following formulae (8) and (9), respectively.
[Math. 14]

$$P(\mathbf{X}) = \prod_{k=1}^{K} P(\bar{\mathbf{x}}_k) = \prod_{k=1}^{K} \mathcal{N}\left(\bar{\mathbf{x}}_k \big| \boldsymbol{\mu}'_k, \boldsymbol{\Sigma}_k^{-1}\right) \tag{8}$$

$$P(\mathbf{r}) = \mathcal{N}(\mathbf{r}|\mathbf{0}, \alpha^{-1}\mathbf{I}) \tag{9}$$

In formula (9), $\alpha$ is a hyperparameter for controlling the degree of variation of the distribution in estimating the ratios of cell types.
[Math. 15]

In the prior distribution P(X), $\mathcal{N}\left(\bar{\mathbf{x}}_k \big| \boldsymbol{\mu}'_k, \boldsymbol{\Sigma}_k^{-1}\right)$ is the same as that in formula (4), wherein $\boldsymbol{\mu}'_k = \frac{1}{N_k}\sum_j \mu_{w_j}\mathbf{x}_j$, and $\boldsymbol{\Sigma}_k^{-1} = \frac{1}{N_k}\sum_j \sigma_{w_j}^2$ diag($x_j\odot x_j$).

Instead of directly maximizing the posterior distribution, an iterative method for estimating P(r|y,X) and $\bar{\mathbf{x}}_k$ with $P(\bar{\mathbf{x}}_k|\mathbf{y}, \mathbf{r}, \{\mathbf{x}\}_{l\neq k})$ maximized for r and $\bar{\mathbf{x}}_k$, respectively, is adopted.

Specifically, for the estimation of $\bar{\mathbf{x}}_k$, a probability formula represented by the following formula (10) is adopted.

$$P(\bar{\mathbf{x}}_k|\mathbf{y}, \mathbf{r}, \{\bar{\mathbf{x}}_l\}_{l\neq k}) \propto P(\mathbf{y}|\mathbf{X}, \mathbf{r})P(\bar{\mathbf{x}}_k). \tag{10}$$

$P(\bar{\mathbf{x}}_k|\mathbf{y}, \mathbf{r}, \{\bar{\mathbf{x}}_l\}_{l\neq k})$ follows a Gaussian distribution, and its mean and variance are calculated according to the following formulae (11) and (12), respectively.

$$\mathbb{E}[\bar{\mathbf{x}}_k] = \left(\beta r_k^2\mathbf{I} + \boldsymbol{\Sigma}_k^{-1}\right)^{-1}\left(\beta r_k\mathbf{y} + \boldsymbol{\Sigma}_k^{-1}\boldsymbol{\mu}'_k - \beta\sum_{l\neq k} r_k r_l\bar{\mathbf{x}}_l\right) \tag{11}$$

$$\mathrm{Var}[\bar{\mathbf{x}}_k] = \beta r_k^2\mathbf{I} + \boldsymbol{\Sigma}_k^{-1}. \tag{12}$$

In the result of $\mathbb{E}[\bar{\mathbf{x}}_k]$, negative values are all set to "0."

For the estimation of r, a probability formula represented by the following formula (13) is adopted.

$$P(\mathbf{r}|\mathbf{y}, \mathbf{X}) \propto P(\mathbf{y}|\mathbf{X}, \mathbf{r})P(\mathbf{r}). \tag{13}$$

P(r|y,X) follows a Gaussian distribution, and its mean and variance are calculated according to the following formula (14).

$$\mathbb{E}[\mathbf{r}] = (\alpha\mathbf{I} + \beta\mathbf{X}^t\mathbf{X})^{-1}\beta\mathbf{X}^t\mathbf{y}. \tag{14}$$

For both results $\mathbb{E}[\mathbf{r}]$ and $\mathbb{E}[\bar{\mathbf{x}}_k]$, negative values are all set to "0." In order to estimate the gene expression patterns $\{\bar{\mathbf{x}}_k^*\}_{k=1}^{K}$) and the composition ratios (r*) of cell types, iterative calculations are alternately performed until both X and r converge or otherwise 1001 iterations are performed.

As initial r and X, the composition ratios of cell types and the counts of the reference data set weighted by the calculation formula (4) are used. For convenience sake, the hyperparameters $\alpha$ and $\beta$ can be set to $10^{-3}$, $10^{-2}$, ..., $10^3$. The result of a combination of the numbers of the hyperparameters ($\alpha$ and $\beta$) of a signature gene set (100 to 2000 genes) that generated high similarity (showed high Pearson and Spearman correlation coefficients and having similarity determined based on a low mean square error) to the real whole-organ RNA-Seq can be selected as an optimum estimation result.

3. Device for correcting count data set for scRNA-Seq analysis, device for analyzing scRNA-Seq, and device for analyzing composition ratios of cell types composing organ to be analyzed 3-1. Device for correcting count data set for scRNA-Seq analysis

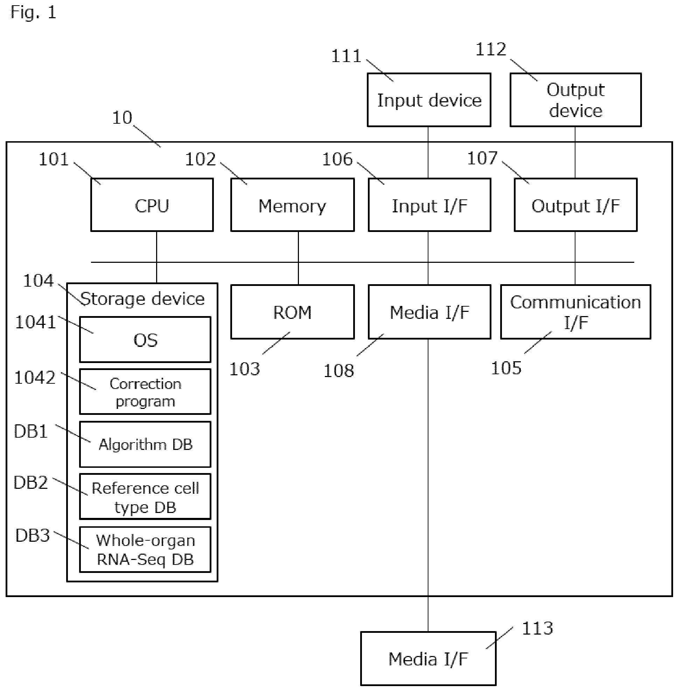

FIG. 1 shows a hardware configuration of a device 10 for correcting a count data set for scRNA-Seq analysis.

The correcting device 10 may be a general-purpose computer. The correcting device 10 is communicably connected to an input device 111, an output device 112, and a media drive 113. The correcting device 10 includes a CPU 101, a memory 102, a ROM (read only memory) 103, a storage device 104, a communication interface (I/F) 105, an input interface (I/F) 106, an output interface (I/F) 107, and a media interface (I/F) 108. The components in the correcting device 10 are connected for mutual data communication by a bus 109.

The storage device 104 is constituted of a hard disk, a semiconductor memory element such as a flash memory, an optical disk or the like. In the storage device 104, an operating system (OS) 1041, a correction program 1042, which is described later, an algorithm database (DB) DB1, a reference cell type database (DB) DB2, and a whole-organ RNA-Seq database (DB) DB3 are stored. The correction program 1042 causes a computer to function as the correcting device 10 in corporation with the operating system 1041.

In this embodiment, the CPU 101 is referred to also as "control part 101."

The algorithm database DB 1 stores the mathematical formulae for performing correction described in Section 1-1-3. above. In the reference cell type database DB2, labels indicating cell types contained in respective organs are stored with their composition ratios and data counts for scRNA-Seq analysis of respective cell types linked therewith. Also, in the reference cell type database DB2, corrected data counts for scRNA-Seq analysis of respective cell types are stored with labels indicating the names of organs and labels indicating the names of cell types linked therewith. In the whole-organ RNA-Seq database DB3, each count data for whole-organ RNA-Seq analysis of mice or humans is registered for each organ. These data items are generated from known data described in Section 1-1-2. and stored.

The input device 111 is constituted of a touch panel, keyboard, mouse, pen tablet, microphone or the like, and performs character input or sound input into the correcting device 10. The input device 111 may be externally connected to the control part 101 or may be integrated with the correcting device 10.

The output device 112 is constituted, for example, of a display device such as a display, a printer or the like, and outputs various operation windows, analysis results and so on.

The media drive 113 may be a USB drive, flexible disk drive, CD-ROM drive, DVD-ROM drive or the like.

The communication I/F 105 communicates with external databases and other computers. The output I/F 107 transmits information to the output device 112.

3-2. Processing by correction program

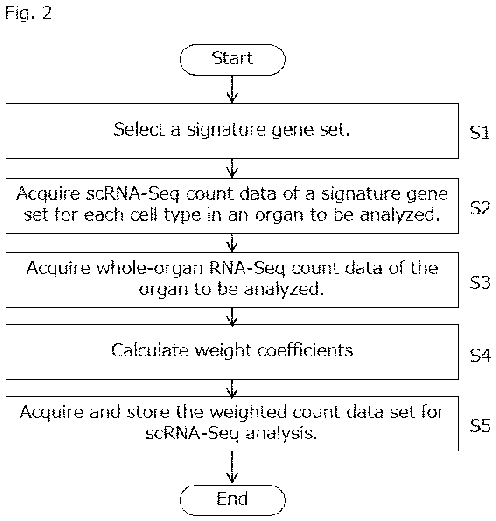

FIG. 2 shows the flow of processing by the correction program 1042.

First, the control part 101 of the correcting device 10 accepts a command to start processing input by an operator through the input device 111, and starts processing. In step S1, the control part 101 selects signature genes that characterize each cell type of organs to be analyzed according to the method described in Section 1-1-2. above.

Next, in step S2, the control part 101 acquires scRNA-Seq count data of a signature gene set acquired in step S1 from the reference cell type database DB2.

Next, in step S3, the control part 101 acquires whole-organ RNA-Seq count data from the whole-organ RNA-Seq database DB3. It should be noted that step S3 may be prior to step S2.

Next, in step S4, the control part 101 reads out formulae (1) to (4) described in Section 1-1-2. above from the algorithm database DB1. The control part 101 calculates weight coefficients for respective cell types present in respective organs based on the formulae described in Section 1-1-2. above by applying the scRNA-Seq count data of a signature gene set acquired in step S2 and the whole-organ RNA-Seq count data acquired in step S4 to each formula read out. The control part 101 stores the calculated weight coefficients in the algorithm database DB1.

Finally, in step S5, the control part 101 acquires a count data set for scRNA-Seq analysis weighted for each cell type according to Section 1-1-3., and stores it in the reference cell type database DB2.

Further, the control part 101 may receive a command to start output processing input by the operator through the input device 111, and output the weighted count data set for scRNA-Seq analysis from the output device 112.

Although an example in which steps S1 to S5 are performed by one computer is shown in this embodiment, step S1, steps S2 to step S4, and step S5, for example, may be performed different computers. In other words, a first computer may select signature genes according to step S1, and a second computer may acquire information about a signature gene set of respective cell types present in respective organs from the first computer and perform the processing in step S2 to step S4 to calculate weight coefficients. Further, a third computer may acquire a weighted count data set for scRNA-Seq analysis.

Further, a first computer may perform step S1 to step S4, and a second computer may perform step S5.

Also, a first computer may perform step S1, and a second computer may perform step S2 to step S5.

3-3. Device for analyzing scRNA-Seq, and device for analyzing composition ratios of cell types composing organ to be analyzed

As described in Section 2. above, analysis of scRNA-Seq and analysis of composition ratios of cell types composing an organ to be analyzed can be performed simultaneously. Thus, an analyzing device 20 performs both processing.

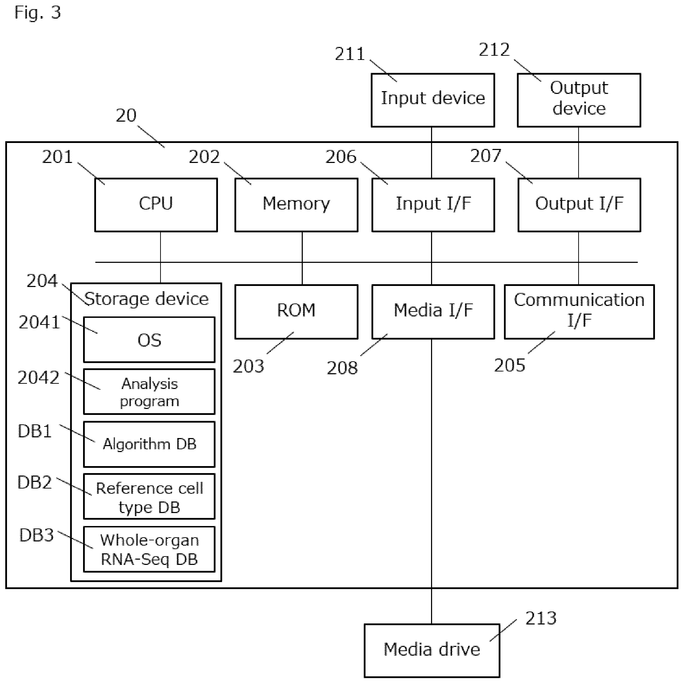

FIG. 3 shows a hardware configuration of the analyzing device 20. The analyzing device 20 basically has the same configuration as the correcting device 10 except a storage device 204. The storage device 204 stores an analysis program 2042, which is described later, in place of the correction program 1042. The storage device 204 further stores an algorithm database (DB) DB1, a reference cell type database (DB) DB2, a whole-organ RNA-Seq database (DB) DB3 similarly to the storage device 104.

3-4. Processing by analysis program

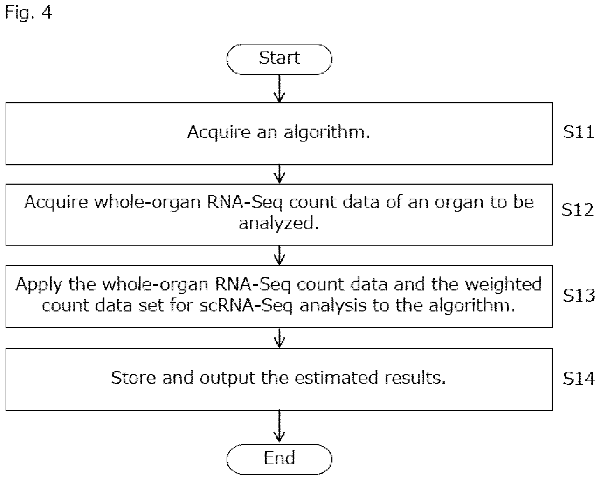

FIG. 4 shows the flow of processing by the analysis program 2042.

First, a control part 201 of the analyzing device 20 accepts a command to start processing input by an operator through an input device 211, and starts processing. In step S11, the control part 201 reads out an algorithm as described in Section 2. above from the algorithm database DB1.

Next, in step S13, the control part 201 acquires whole-organ RNA-Seq count data from the whole-organ RNA-Seq database DB3.

Subsequently, in step S13, the control part 201 reads out the weighted count data set for scRNA-Seq analysis acquired in Section 3-2. above from the reference cell type database DB2 and applies it to the algorithm.

Next, the control part 201 records the composition ratios of cell types composing an organ to be analyzed estimated by the algorithm and estimated count data for scRNA-Seq analysis in the storage device 204 as estimation results.

As an estimate result, the control part 201 may output only the composition ratios of cell types composing an organ to be analyzed from an output device 212 or may output only the estimated count data for scRNA-Seq analysis from the output device 212. Also, the control part 201 may output both the results from the output device 212.

4. Recording medium having computer program recorded therein

The correction program 1042 and the analysis program 2042 may be recorded in a recording medium.

In other words, each program is stored in a recording medium such as a hard disk, a semiconductor memory element such as a flash memory, an optical disk or the like. Also, each program may be stored in a recording medium connectable via a network such as a cloud server. Each program may be provided as a program product in a downloadable form or recorded in a recording medium.

The storage format of the programs in the recording medium is not limited as long as each of the devices can read the programs. The storage in the recording medium is preferably in a nonvolatile manner.

Examples

Examples are shown below to describe the present invention in more detail. However, the present invention should not be construed as being limited to the examples.

I. Methods

1. Calculation of composition ratios of reference cell types

Based on scRNA-Seq data described in Non-Patent Document 5 and databases registered in NIH and so on, the composition ratios of reference cell types were calculated for the following 14 organs; aorta, brain, fat, heart, kidney, large intestine, liver, lung, bone marrow, pancreas, skin, skeletal muscle, spleen and thymus.

For aorta, fat, kidney, large intestine, liver, lung, bone marrow, pancreas, skin, spleen and thymus, the ratios of cell types in each organ described in Non-Patent Document 5 were used as the composition ratios of reference cell types.

For skeletal muscle, the ratios of cell types described in Non-Patent Document 2 were used as the composition ratios of reference cell types.

For heart, the composition ratios of cell types described in Non-Patent Document 5 were corrected in connection with the separation analysis between cardiac muscle cells and non-muscle cells and used as the composition ratios of reference cell types (References). Specifically, the composition ratio (3.1%) of cardiac muscle cells adopted in Non-Patent Document 5 is extremely low compared to the ratios (30% to 40%) that have been generally consented based on various previous studies in the field of histological anatomy. Thus, in this example, the ratio of cardiac muscle cells was set to 30%, and the composition ratios of reference cell types were obtained by dividing the remaining 70% by the composition ratios of non-muscle cell types.

For brain, the composition ratios of reference cell types were determined based on a report in NIH (http://www.nervenet.org/papers/BrainRev99.html#Numbers). As labels indicating respective cell types in brains, corresponding cell type labels of scRNA-Seq data described in Non-Patent Document 5 were used. First, the cell type classes in brains were classified into four classes; "neuron," "glial cells," "endothelial cells" and "others," and the ratios of respective classes were set to 75:23:7:4. The ratios were determined according to the estimated ratios in brains of mice (http://www.nervenet.org/papers/BrainRev99.html#Numbers). Next, according to Non-Patent Document 5, the classes of "neuron," "glial cells" and "others" were classified into more detailed cell type classes. Specifically, the class of "neuron" was further classified into "nerve cells-excitable neurons and several neural stem cells" and "nerve cells-inhibitory neurons." The class of "others" was classified into "brain pericytes-NA" and "oligodendrocyte precursor cells-NA." The class of "glial cells" was classified based on the following three premises, i) The class of "glial cells" can be classified into four cell types according to Non-Patent Document 5; "microglial cells-NA," "astrocytes-NA," "Bergmann glial cells-NA" and "oligodendrocytes-NA." ii) The composition ratios of these four glial cell types follow the description in Non-Patent Document 5. iii) Because "microglial cells" are reported to account for 10 to 15% of cells in the whole brain, the ratio of "microglial cells-NA" in the whole brain is set to 0.1. Based on these premises, the rates of respective brain cell types were set to as follows; "macrophages-NA" (approximately 0.2%), "microglial cells-NA" (10.0%), "astrocytes-NA"(approximately 2.2%), "Bergmann glial cells-NA"(approximately 2.1%), "brain pericytes-NA" (approximately 1.5%), "endothelial cells-NA" (approximately 6.4%), "nerve cells-excitable neuron and several neural stem cells" (approximately 47.5%), "nerve cells-inhibitory neurons "(approximately 21.3%), "oligodendrocytes-NA" (approximately 8.7%), and "oligodendrocyte precursor cells-NA" (approximately 1.9%). These were used as the composition ratios of reference cell types of brain. For human heart and kidney, the composition ratios of cell types in the hearts of mice and the composition ratios of cell types in the kidneys of mice were used as the composition ratios of reference cell types.

The composition ratios of reference cell types in each organ are shown in the list of composition ratios of reference cell types, which is described later.

Also, for each cell type shown in the list of composition ratios of each reference cell type, count data for scRNA-Seq is registered for each cell type in known databases.

2. Preprocessing of data and normalization of RNA counts

Data processing and analysis were all conducted using software "R" version 3.6.1. All cell type labels were the same as the labels attached in previously reported scRNA-Seq studies. The gene symbols attached to the scRNA-Seq data were subjected to association conversion to each item of whole-organ RNA-Seq data by entrez gene IDs derived from "org.Mm.egALIAS2EG" in the R package of"org.Mm.eg.db." The genes with an ERCC label attached thereto were deleted because they are spike-in genes. Further, the RNA counts derived from three genes Rn45s, Akap5 and Lrrc17 were also deleted because they are non-mRNA artifacts that significantly affect the total count. Next, the RNA count derived from each gene was normalized by converting it such that the total count of each cell in the scRNA-Seq data set is 100. This normalization step was performed in the same manner on each RNA included in the whole-organ RNA-Seq data set.

3. Selection of signature gene set for identification of cell types

Using random forest (RF), signature genes of each cell type were selected with a computer using the composition ratio data set of reference cell types and scRNA-Seq data described in the previous session. In this selection, the "randomForest" package of R was used for the tuning and creation of a classifier by RF. The scRNA-Seq data was first divided into two parts, and one was used as training data for creating a classifier by RF and the other was used as test data for calculation of F1 scores to verify the accuracy of the classifier. RF analysis was performed with a data set in which the composition ratios of cell types were maintained as described in the previous session. Following the creation of a classifier, important feature amounts of the classifier were extracted as the names of signature genes of each cell type, and a "Mean Decrease Gini" value was used as an importance index of each gene.

4. Database

Data sets used in this example were all disclosed. The whole-organ RNA-Seq data of mice and the whole-organ RNA-Seq of myocardial infarction model mice were acquired from "i-organs.atr.jp." The human whole-organ RNA-Seq data was acquired from "The Human Protein Atlas" (https://www.proteinatlas.org/; heart (ERR315328) and kidney (ERR315494)). The scRNA-Seq data was acquired from Non-Patent Document 5 (aorta, brain, fat, heart, kidney, large intestine, liver, lung, bone marrow, pancreas, skin, spleen and thymus), and Skeletal Muscle of "Mouse Cell Atlas."

5. Estimated gene expression variation analysis

The total of the RNA counts of all genes predicted and calculated was normalized to one million copies. The normalized count of each gene was rounded to the nearest integer and analyzed using an R package "DESeq2 (version 1.24.0)."

II. Validation of performance of previously reported whole-organ RNA-Seq data deconvolution methods

First, for the MuSiC method (Non-Patent Document 17) and the DWLS method (Non-Patent Document 19) as previously reported methods, a side-by-side comparison was performed on the composition ratios of cell types calculated by respective methods and the composition ratios of reference cell types obtained from the scRNA-Seq data and reports in the past to verify the performance of each deconvolution method.

1. Calculation of composition ratios of cell types by previously reported methods

The MuSiC method (Non-Patent Document 17) and the DWLS method (Non-Patent Document 19) were performed according to each document. When a quadratic problem solver was performed by the DWLS method, solve.QP (R package: quadprog) was replaced with solve_osqp (R package: osqp).

2. Results

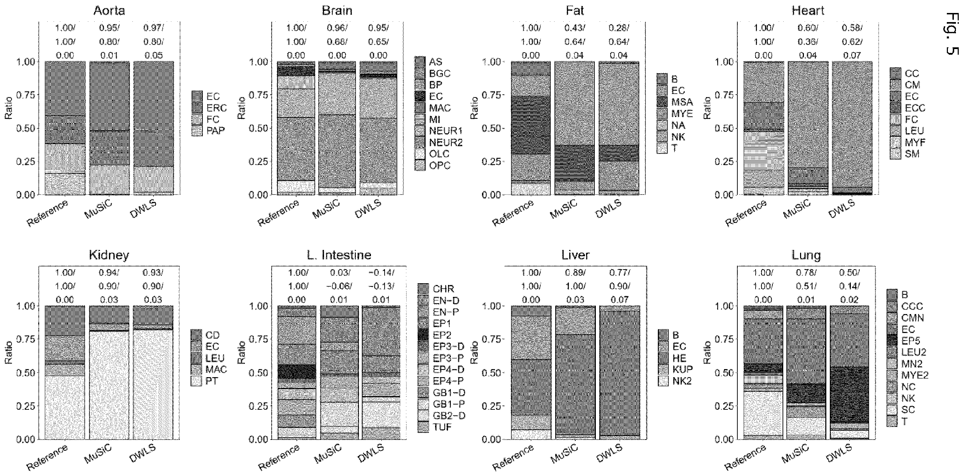

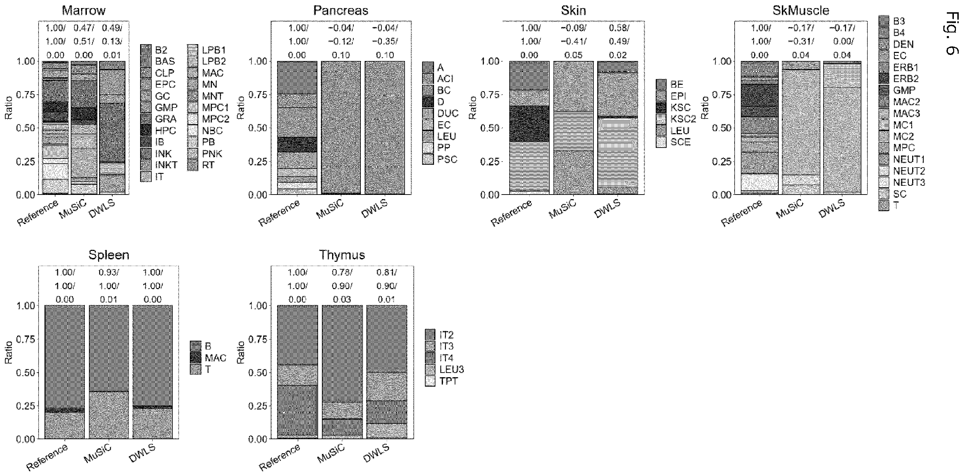

FIG. 5 and FIG. 6 show the results of comparison between the estimated composition ratios of cell types in each organ calculated by a computer using the MuSiC or DWLS method and the composition ratios of reference cell types in each organ prepared in Section 2. above. The estimated composition ratios of cell types in each organ estimated by the MuSiC or DWLS method deviated from the composition ratios of reference cell types, and the degree of deviation also varied. In particular, the deviations were pronounced for skeletal muscle, heart, pancreas and liver.

A heart is composed of cardiac muscle cells and non-muscle cells. The cardiac muscle cells account for the largest volume of the heart. However, when the numbers of cells are compared, there are more non-muscle cells than cardiac muscle cells. Contrary to this fact, in the composition of cell types in heart calculated by the MuSiC or DWLS method, cardiac muscle cells were calculated to account for 90%. The same tendency was observed for skeletal muscle.

One possible reason for the deviation between the composition ratios of reference cell types and estimated composition ratios of cell types as described above was the difference in total RNA content between different cell types. It has been reported that the total RNA content is different in different cells in the range of 50,000 transcripts/cell to 300,000 transcripts/cell. In the heart, the volume of cardiac muscle cells is said to be 20 to 25 times the volume of non-muscle cells such as endothelial cells and fibroblasts. Thus, the total RNA content per cell can vary largely between muscle cells and non-muscle cells. In fact, this possibility is not taken into account in the MuSiC and DWLS methods. It is considered that such a point led to the deviation between the composition ratios of reference cell types and the estimated composition ratios of cell types.

III. Verification of reason for deviation between estimated whole-organ RNA-Seq data set and real whole-organ RNA-Seq data set

A hypothesis that the deviation between the composition ratios of reference cell types and estimated composition ratios of cell types is due to the difference in total RNA content between respective cell types contained in the tissue collected from the organ when a total RNA sample of the organ is extracted was made, and the hypothesis was verified by comparing a real gene expression profile with an estimated gene expression profile. The estimated whole-organ RNA-Seq data is the results of multiplying the composition ratios of reference cell types acquired in Section 1.1 by the count data acquired in Section 1.2.

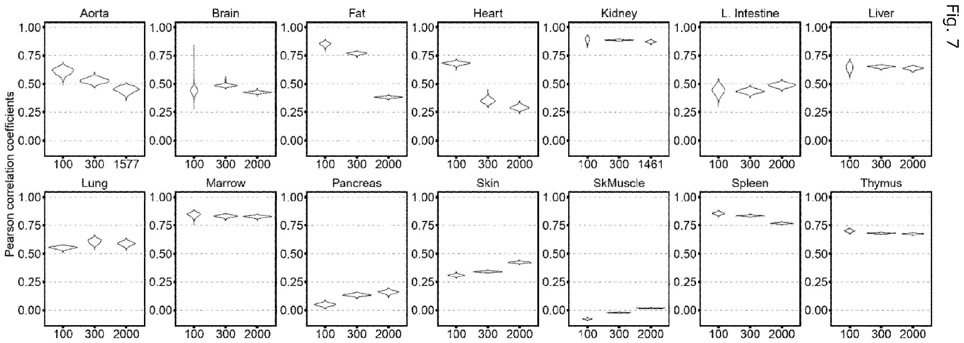

FIG. 7 shows the estimated whole-organ RNA-Seq data. The estimated whole-organ RNA-Seq data was calculated as the sum of transcripts counts for each gene normalized by weighting tissues composed of multiple cell types based on the composition ratios of known reference cell types.

The results shown in FIG. 7 are the indicated number (number of genes) of signature genes calculated by RF according to the number of top ranks in each cell types in each organ used to identify the cell types in each organ. The number of top ranks was set to 100 genes, 300 genes and 2000 genes in the signature genes. However, for aorta and kidney, because the total number of signature genes are less than 2000, comparison was made using 1577 genes for aorta and 1461 genes for kidney instead of 2000 genes. The similarity/dissimilarity between the real and estimated gene expression profiles of the 14 organs is shown by Pearson correlation coefficients.

As shown in FIG. 7, for ten organs (aorta, brain, heart, large intestine, liver, lung, pancreas, skin, skeletal muscle and thymus) the Pearson correlation coefficient was less than 0.75. This indicates that simply multiplying the composition ratios of reference cell types acquired in Section 1.1 by the count data acquired in Section 1.2. is not sufficient for these organs to reconstruct whole-organ RNA-Seq data.

IV. Setting and verification of cell type-specific weight coefficients to eliminate deviation between data sets

Weight coefficients for correcting the RNA contents in different cell types present in each tissue were calculated and their accuracy was verified.

1. Calculation of cell type-specific coefficients

Weight coefficients for respective cell types present in respective organs were calculated according to the following method.

[Math. 16] $\mathbf{y} \in \mathbb{R}^{n}, w_{j} > 0$, $w_j > 0$, and $\mathbf{x}_{j} \in \mathbb{R}^{n}$ represent a vector of normalized whole-organ RNA-Seq counts, a weight coefficient for each cell j to be analyzed, and a matrix of normalized scRNA-Seq counts, respectively. Here, n represents the number of genes of signature genes in each organ. According to the ranking based on "Mean Decrease Gini" obtained by RF analysis, the top 100, 300 or 2,000 genes were selected as signature genes. For organs with a maximum number of signature genes less than 2000, all genes were used in RF analysis. In addition, a combination $C^m$ of cells to be analyzed was randomly selected under the restriction that the composition ratios of reference cell types are kept within a total set size m.

Next, the following formula (1) is described.
Math. 17]

$$my = \sum_{j} w_{j}\mathbf{x}_{j} \ (j \ \in \ C^{m}) \tag{1}$$

In formula (1), m represents a multiplying factor, which is determined depending on n. m is set to a value smaller than n in each of the following calculations. Here, $w_j$ was calculated by solving a quadratic programming problem according to the formula (2) below under the restriction that the resulting value is 0.01 or greater.
[Math. 18]

$$\widetilde{w}_{j} = \mathrm{argmin}_{w_{j}} \sum_{i}^{S} \left| m\mathbf{y}_{i} - \sum_{j} w_{j}\mathbf{x}_{j} \right|^{2} \ s.t. \ w_{j} \geq 0.01. \tag{2}$$

In formula (2), S represents the number of count data sets for RNA-Seq for each gene targeting the whole organ. In this study, corresponding count data sets for whole-organ RNA-Seq acquired from two different individuals were used. Therefore, S is 2. This quadratic programming problem was solved in R using a "quadprog" package. The both steps of randomly selecting combinations of cells to be analyzed and calculating

[Math. 19] $\tilde{w}_j$ were recursively done for all the selected cells to be analyzed until the number of $w_j$ reached 100 or more.

Next, on the premise that the distribution of weight coefficients follows a Gaussian distribution, the mean and variance of weighted counts of genes of respective cells to be analyzed were calculated according to the following formula (3).

[Math. 20]

$$\tilde{\mathbf{x}}_j \sim \mathcal{N}\left(\mu_{w_j}\mathbf{x}_j, \sigma^2_{w_j}\text{diag}(\mathbf{x}_j \odot \mathbf{x}_j)\right) \tag{3}$$

In formula (3),

[Math. 21]
$$\tilde{\mathbf{x}}_j, \mu_{w_j}, \text{ and } \sigma^2_{w_j}$$

represent a weighted count vector of genes of the cells j to be analyzed, the mean of weight coefficients for cells j to be analyzed, and the variance of weight coefficients for cells j to be analyzed, respectively.

[Math 22] An operator $\odot$ represents an element-wise product between two vectors.

Based on the calculated mean and variance of weight coefficients of respective cells to be analyzed, the mean and variance of weight coefficients in the corresponding cell types were calculated according the following formula (4) on the premise that the mean and variance of weight coefficients in the corresponding cell types follow a Gaussian distribution.

[Math. 23]

$$\bar{\mathbf{x}}_k \sim \mathcal{N}\left(\frac{1}{N_k}\sum_j \mu_{w_j}\mathbf{x}_j, \frac{1}{N_k}\sum_j \sigma^2_{w_j}\text{diag}(\mathbf{x}_j \odot \mathbf{x}_j)\right) \ (j \in C^k). \tag{4}$$

In formula (4), k, $C^k$ and $N_k$ represent a cell type, the group of cells to be analyzed labeled to the cell type k, and the number of cells to be analyzed in $C^k$, respectively.

The weight coefficients for respective cell types present in respective organs, and mean, variance and quartiles thereof calculated according to the above formula (2) are shown in the weight coefficient list described later.

2. Results

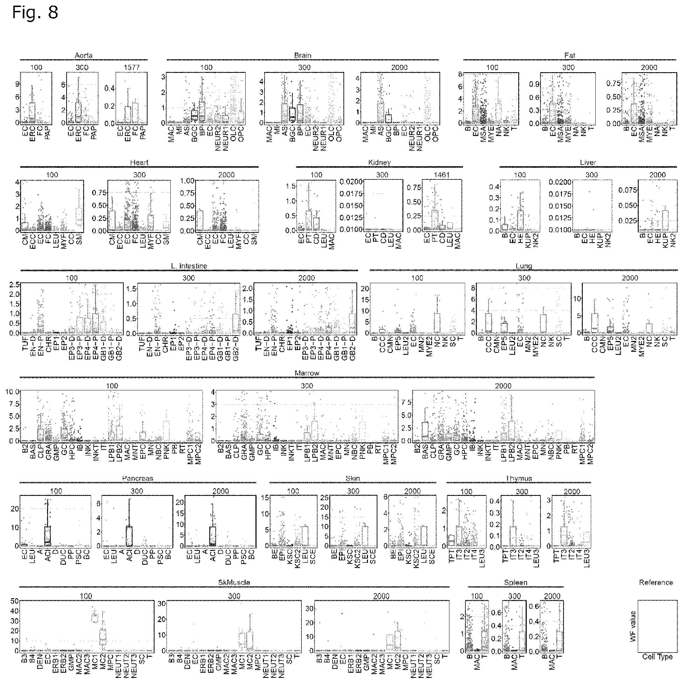

By the above calculation formula (2), weight coefficients of respective cell types and their ranges were created (FIG. 8). The weight coefficient for muscle cells were really greater than that for non-muscle cells for both heart and skeletal muscle (FIG. 8). These cell type-specific weight coefficients were used to weight the transcript counts of respective cell types. Next, according to the composition ratios of reference cell types of respective cell types contained in each organ, the composition ratios of reference cell types of respective cell types in each organ were applied to the transcript counts weighted by the weight coefficients to generate an RNA-Seq data set. This calculation method is referred to as "estimated whole-organ RNA-Seq (v-RNA-Seq)," and an RNA-Seq data set obtained by the estimated whole-organ RNA-Seq is referred to as "estimated whole-organ RNA-Seq data set."

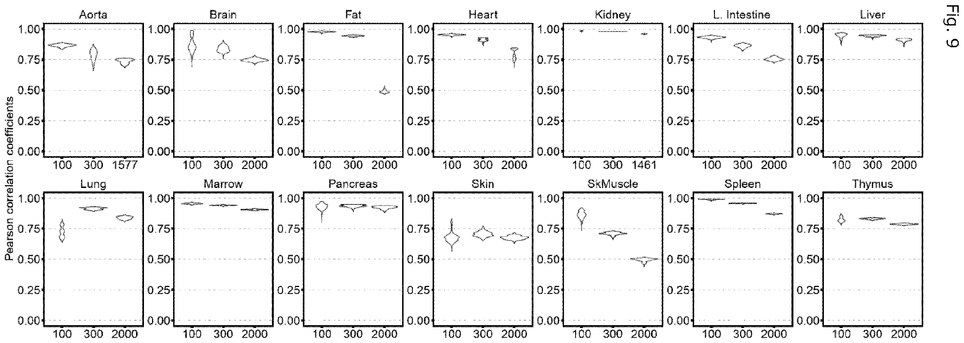

Next, the estimated whole-organ RNA-Seq data set and the corresponding real whole-organ RNA-Seq data set were compared. The results are shown in FIG. 9. Compared to FIG. 7, the deviation of gene expression profiles shown by each data set was reduced for most organs (Pearson correlation coefficients = 0.8-1.0).

V. Calculation of composition ratios of cell types in each organ and estimation of total RNA expression patterns of cell types contained in each organ

Using the specific weight coefficients based on RNA contents of respective cell types calculated in Section IV. above, an algorithm based on the Bayes' theorem was designed, and both the ratios of respective cell types contained in each organ and the gene expression patterns in the respective cell types were simultaneously calculated.

1. Calculations of composition ratios and gene expression patterns of cell types

The composition ratios and gene expression patterns of cell types were calculated according to the following formula (5). The mean and variance of transcript counts weighted by the weight coefficients in each cell type were calculated according to formula (4) above.
[Math. 24]

$$\mathbf{y} = \mathbf{Xr} \tag{5}$$

In formula (5),

[Math. 25]
$$\mathbf{y}, \mathbf{X} = (\bar{\mathbf{x}}_1, \dots, \bar{\mathbf{x}}_k, \dots, \bar{\mathbf{x}}_K),$$

*and* **r**

represent a whole-organ RNA-Seq data vector, a matrix including estimated scRNA-Seq data, which are weighted counts calculated using weight coefficients for respective cell types according the above formula, in its columns, and a coefficient vector corresponding to the composition ratios of cell types, respectively. For the calculation of X and r, the Bayes' theorem was used. In order to apply the Bayes' theorem,

[Math. 26] noise $\mathcal{N}(\mathbf{0}, \beta^{-1}\mathbf{I})$
was added to formula (5), and a probabilistic model shown in the following formula (6) was adopted.
[Math. 27]

$$P(\mathbf{y}|\mathbf{X}, \mathbf{r}) = \mathcal{N}(\mathbf{y}|\mathbf{Xr}, \beta^{-1}\mathbf{I}) \tag{6}$$

In formula (6), β represents a hyperparameter. According to the Bayes' theorem, the posterior distributions of X and r were obtained as the following formula (7).
[Math. 28]

$$P(\mathbf{X}, \mathbf{r}|\mathbf{y}) \propto P(\mathbf{y}|\mathbf{X}, \mathbf{r})P(\mathbf{X})P(\mathbf{r}) \tag{7}$$

In formula (7), P(X) and P(r) represent prior distributions of X and r, respectively. P(X) and P(r) are given as the following formulae (8) and (9), respectively.
[Math. 29]

$$P(\mathbf{X}) = \prod_{k=1}^{K} P(\bar{\mathbf{x}}_k) = \prod_{k=1}^{K} \mathcal{N}(\bar{\mathbf{x}}_k | \boldsymbol{\mu}'_k, \boldsymbol{\Sigma}_k^{-1}) \tag{8}$$

$$P(\mathbf{r}) = \mathcal{N}(\mathbf{r}|\mathbf{0}, \alpha^{-1}\mathbf{I}) \tag{9}$$

In formula (9), α is a hyperparameter. In the prior distribution P(X), $\mathcal{N}(\bar{\mathbf{x}}_k | \boldsymbol{\mu}'_k, \boldsymbol{\Sigma}_k^{-1})$ is the same as that in formula (4), wherein $\boldsymbol{\mu}'_k = \frac{1}{N_k}\sum_j \mu_{w_j}\mathbf{x}_j$ , and $\boldsymbol{\Sigma}_k^{-1} = \frac{1}{N_k}\sum_j \sigma_{w_j}^2$ diag($\mathbf{x}_j \odot \mathbf{x}_j$).

Instead of directly maximizing the posterior distribution, an iterative method for estimating P(r|y,X) and $\bar{\mathbf{x}}_k$ with P($\bar{\mathbf{x}}_k$|y,r,$\{\mathbf{x}_l\}_{l\neq k}$) maximized for **r** and $\bar{\mathbf{x}}_k$, respectively, is adopted.

Specifically, for the estimation of $\bar{\mathbf{x}}_k$, a probability formula represented by the following formula (10) is adopted.

$$P(\bar{\mathbf{x}}_k|\mathbf{y}, \mathbf{r}, \{\bar{\mathbf{x}}_l\}_{l \neq k}) \propto P(\mathbf{y}|\mathbf{X}, \mathbf{r})P(\bar{\mathbf{x}}_k). \tag{10}$$

$P(\mathbf{x}_k|\mathbf{y},\mathbf{r}, \{\bar{\mathbf{x}}\}_{l \neq k})$ follows a Gaussian distribution, and its mean and variance were calculated according to the following formulae (11) and (12), respectively.

$$\mathbb{E}[\bar{\mathbf{x}}_k] = \left(\beta r_k^2 \mathbf{I} + \Sigma_k^{-1}\right)^{-1}\left(\beta r_k \mathbf{y} + \Sigma_k^{-1}\mu_k' - \beta \sum_{l \neq k} r_k r_l \bar{\mathbf{x}}_l\right) \tag{11}$$

$$\mathrm{Var}[\bar{\mathbf{x}}_k] = \beta r_k^2 \mathbf{I} + \Sigma_k^{-1}. \tag{12}$$

In the result of $\mathbb{E}[\bar{\mathbf{x}}_k]$ , negative values were all set to "0."

For the estimation of r, a probability formula represented by the following formula (13) was adopted.

$$P(\mathbf{r}|\mathbf{y}, \mathbf{X}) \propto P(\mathbf{y}|\mathbf{X}, \mathbf{r})P(\mathbf{r}). \tag{13}$$

$P(\mathbf{r}|\mathbf{y},\mathbf{X})$ follows a Gaussian distribution, and its mean and variance were calculated according to the following formula (14).

$$\mathbb{E}[\mathbf{r}] = (\alpha\mathbf{I} + \beta\mathbf{X}^t\mathbf{X})^{-1}\beta\mathbf{X}^t\mathbf{y}. \tag{14}$$

For both results $\mathbb{E}[\mathbf{r}]$ and $\mathbb{E}[\bar{\mathbf{x}}_k]$ , negative values were all set to "0." In order to estimate the gene expression patterns ( $\{\bar{\mathbf{x}}_k^*\}_{k=1}^K$ ) and the composition ratios ($\mathbf{r}^*$) of cell types, iterative calculations were alternately performed until both X and r converge or otherwise 1001 iterations were performed.

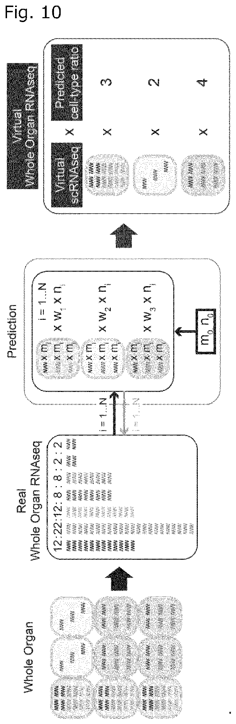

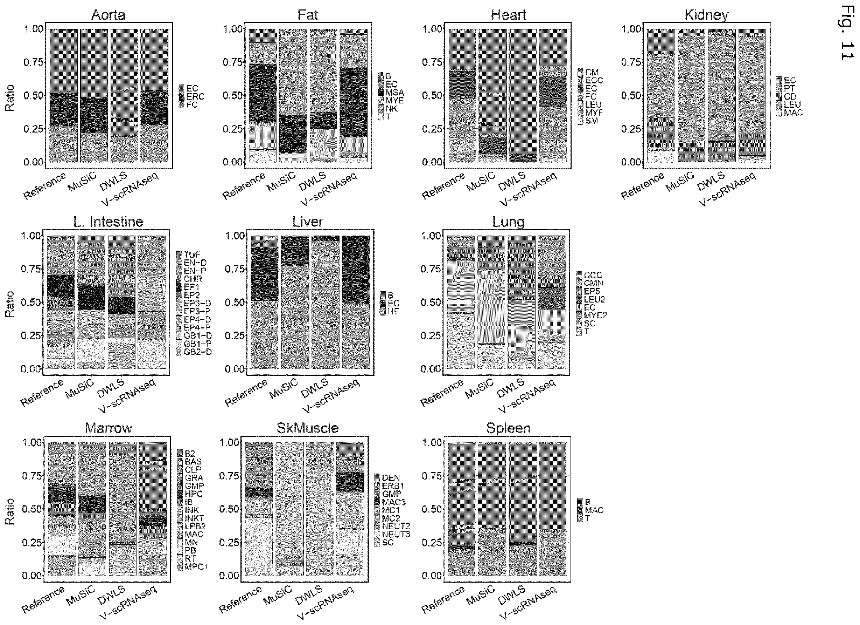

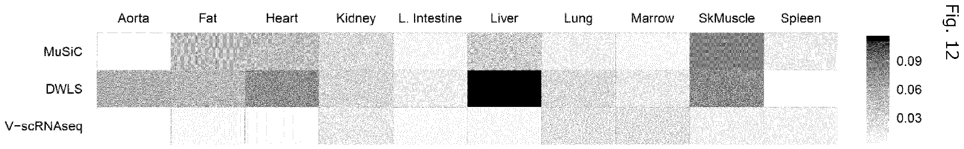

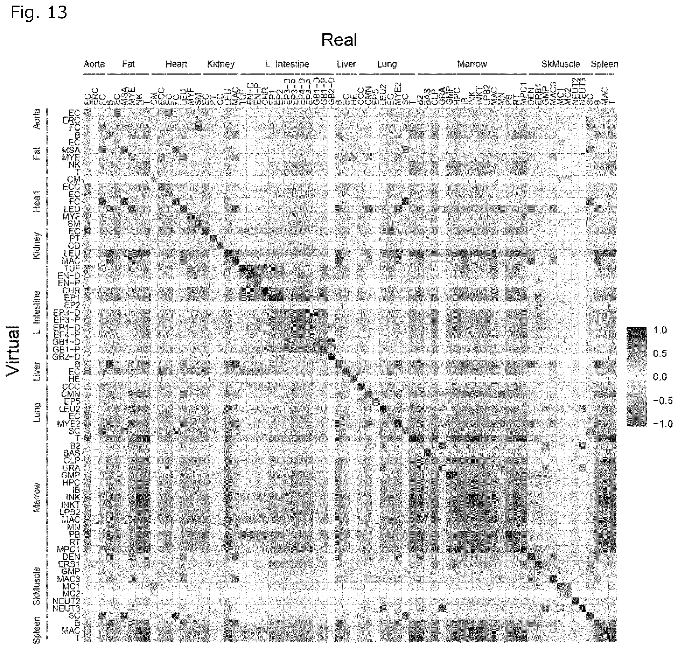

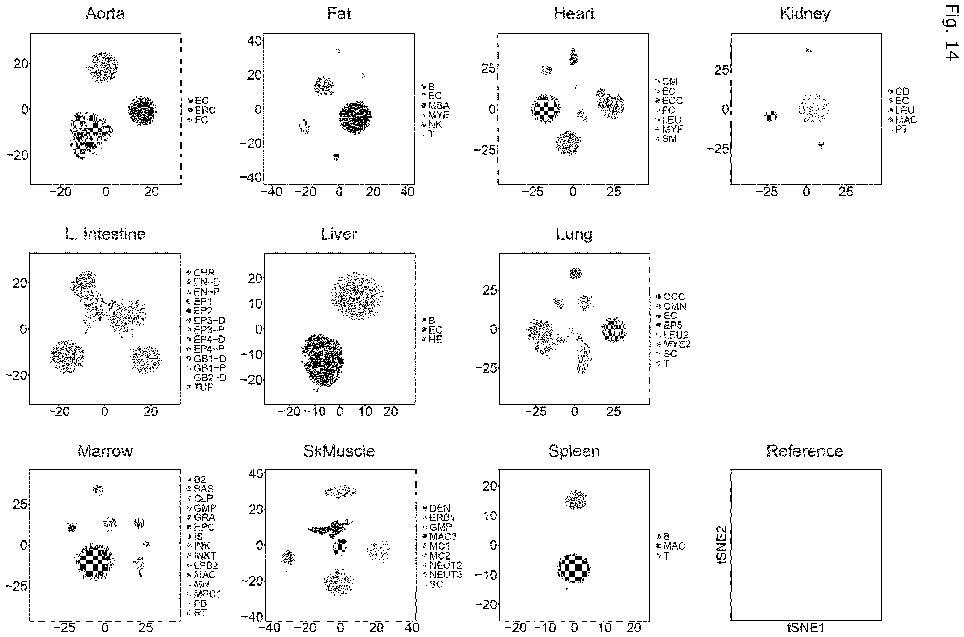

As initial r and X, the composition ratios of cell types and the counts of the reference data set weighted by the calculation formula (4) were used. For convenience sake, the hyperparameters $\alpha$ and $\beta$ were set to $10^{-3}$, $10^{-2}$, ..., $10^3$. The result of a combination of the numbers of the hyperparameters ($\alpha$ and $\beta$) of a signature gene set (100, 300, 2,000/1,577/1,461) that generated high similarity (showed high Pearson and Spearman correlation coefficients and having similarity determined based on a low mean square error) to the real whole-organ RNA-Seq was selected as an optimum estimation result. The overview of this calculation is shown in FIG. 10. The results of comparison between the composition ratios of cell types estimated by the method of the present invention and the ratios of reference cell types are shown in FIG. 11 and FIG. 12. Also, the results of comparison of scRNA-Seq count data estimated by the method of the present invention with real scRNA-Seq, and the results of t-Distributed Stochastic Neighbor Embedding (t-SNE) analysis are shown in FIG. 13 and FIG. 14, respectively.

2. Verification of cell type identification

t-Distributed Stochastic Neighbor Embedding (t-SNE) was used to verify whether the estimated scRNA-Seq count data calculated in Section V.1. above can identify cell types present in each organ. The total sampling size was set to 3,000 for cells belonging to respective cell types present in respective organs, and the number of cells sampled from each cell type and the estimated scRNA-Seq count data of a cell type k were set to

[Math. 30]
$$P^*(\mathbf{r}) \text{ and } P^*(\bar{\mathbf{x}}_k),$$

respectively. This sampling process was repeated until the total sampling size reached 3,000. Next, an R package "Rtsne" was used to apply t-SNE to the sampled estimated scRNA-Seq count data with a parameter perplexity = 50.

3. Results

In the present invention, two hyperparameters $\alpha$ and $\beta$ were defined to take into account the effect of the combination of cell type ratios. The gene expression patterns at different organ levels, for example, the gene expression patterns in normal and pathological organs may be different. However, there are two possible cases for this difference; i) a case where the gene expression pattern in each cell type is apparently the same but the ratios of respective cell types are different, and ii) a case where the ratios of cell types are the same but there are differences in gene expression pattern among the same cell types. Also, there is a possibility that i) and ii) are combined. Therefore, in order to evaluate comprehensive combinations of a wide range of $\alpha$ and $\beta$ to describe the behavior of transcriptome at organ levels, an optimum combination of the composition of cell types and weighted transcriptome counts for each cell type was calculated.

By this method, composition ratios of cell types in ten organs (aorta, fat, heart, kidney, liver, lung, large intestine, bone marrow, skeletal muscle and spleen) were calculated. The results are shown in FIG. 11. From the 14 organs used in FIG. 5 and FIG. 6, brain, pancreas, skin and thymus were excluded from the study for the following reasons. 1) The real ratios of cell types are not available. 2) Pancreas is really derived from pancreatic islet. The real ratios of cell types can be used for pancreatic islet, but they do not represent the real ratios of the entire pancreas. 3) For skin or thymus, the Pearson correlation coefficients did not exceed 0.8 even when cell type-specific weight coefficients were used.

The composition ratios of cell types calculated for the above ten organs were similar to the real composition ratios of reference cell types experimentally determined by scRNA-Seq studies (FIG. 11). In particular, the abnormally large ratios of cardiac muscle cells and skeletal muscle cells estimated by the MuSiC and DWLS methods were both improved by V-scRNA-Seq. The results are shown in FIG. 11. Also, as shown in FIG. 12, for the mean square errors (MSEs) relative to the composition ratios of reference cell types, V-scRNA-Seq was outperformed the other methods for five real organs (fat, heart, large intestine, liver and skeletal muscle).

Also, for 23,131 genes expressed in any of examined organs except for skeletal muscle and 14,323 (skeletal muscle) genes of skeletal muscle included in the estimated whole-organ RNA-Seq data set, estimated transcript counts corrected with cell type-specific weight coefficients and the composition ratios of reference cell types were calculated according to the method of the present invention, and the corrected estimated transcript counts were compared with the real gene expression in respective cell types in the ten organs.

The Pearson correlation coefficients showed that the estimated transcript counts are comparable to the real counts for all cell types and organs (FIG. 13). Also, similarity and relevance of annotations of the same or related cell types among different organs were shown (FIG. 13).

t-SNE analysis using V-scRNASeq data of all the ten organs showed that each cell type can be classified according to the gene expression profile in all the respective organs (FIG. 14).

VI. Calculation of changes in cell type ratios and gene expression in diseases

Next, it was evaluated whether or not our method can detect changes in cell type ratios of respective cell types and gene expression associated with a disease process. Cardiovascular disease is the world's leading cause of death (https://www.who.int/news-room/fact-sheets/detail/cardiovascular-diseases-(cvds)). There are reports that show changes in cell type composition over time during heart disease. Further, as mentioned above, heart is an organ for which both the composition ratios of cell types and their gene expression patterns can be effectively calculated not by a previously disclosed deconvolution method but by the method according to the present invention. Thus, the method of the present invention was applied to mouse models with myocardial infarction (MI) to examine whether or not the method according to the present invention can detect both the composition ratios of cell types in heart and the already known changes over time in cell type-dependent gene expression during MI.

For disease model calculation, weight coefficients were first calculated using whole-organ RNA-Seq data of sham hearts and using the composition ratios of the same reference cell types of normal mice at each stage (E, M, L). Next, using whole-heart RNAs-Seq data from sham/MI models, the composition ratios of cell types and gene expression profile at each stage were calculated as described above.

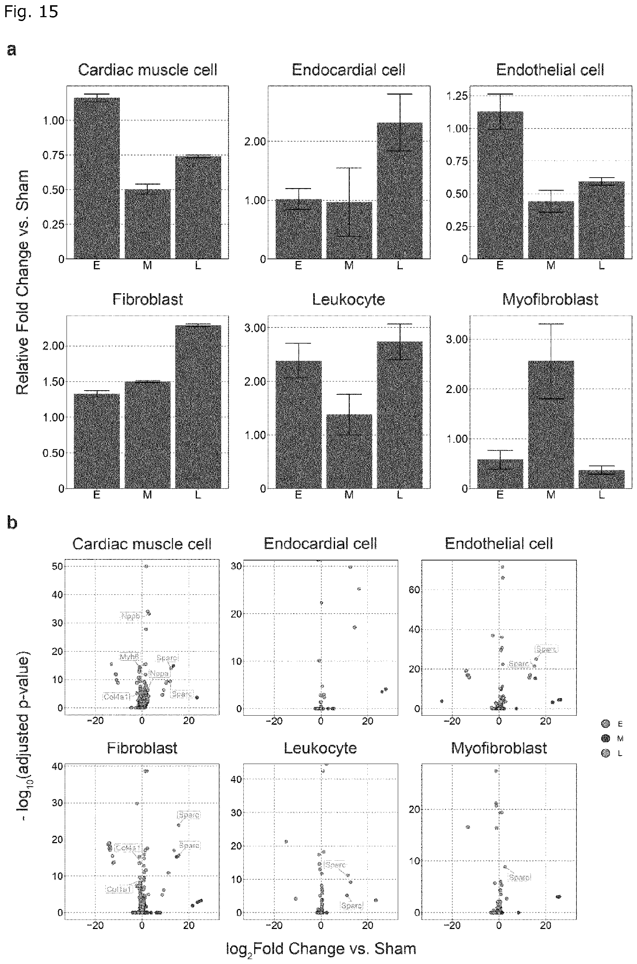

FIG. 15 shows the results.

The method for creating animal models with myocardial infarction is known. The three stages of myocardial infarction are as follows: 1) One day after coronary artery ligation (E-MI, early myocardial infarction stage), 2) Seven days after coronary artery ligation (M-MI, early fibrosis stage) and 3) Eight weeks after coronary artery ligation (L-MI, cardiac remodeling stage). In this analysis, RNA-Seq data of sham controls (E-sham, M-sham and L-sham) and the composition ratios of reference cell types in normal mouse hearts were used to calculate weight coefficients for respective cell types.

By the operation, two expected changes in cell type composition related to MI, specifically, a decrease of cardiac muscle cells and an increase of fibroblasts, were detected (FIG. 15a). By this method, an increase of myofibroblasts, which is characteristic during the M-MI stage, was detected (FIG. 15a). This was also consistent with reported experimental results.

The changes in gene expression in each cell type during myocardial infarction calculated by the present invention led to detection of multiple features expected from previous experimental studies (FIG. 15b).

In cardiac muscle cells, statistically significant increased expression of Nppb, Sparc and Col4a1 genes ($\log_2$ fold change > 0.7), and decreased expression of Myh6 gene ($\log_2$ fold change < 0.7) were detected (FIG. 15b). In fibroblasts, statistically significant increased expression of Col4a1, Col1a1 and Sparc genes ($\log_2$ fold change > 0.7) was detected (FIG. 15b). Here, "statistically significant" means that the adjusted p value < 0.001. In addition to these known landmark genes in MI pathology, many other genes that vary in expression in each cell type depending on the pathology were found by this method (FIG. 15b).

VII. Verification of estimated human scRNA-Seq

Applicability of the weight coefficients and V-scRNA-Seq for mice to deconvolution of a human whole-organ RNA-Seq data set was verified. Publicly available human whole-organ RNA-Seq data for heart and kidney was used to calculate the composition ratios and transcriptome profiles of those cell types.

First, each of total RNA counts expressed from each gene stored in the human whole-organ RNA-Seq data set was normalized to 100. Next, by extracting genes with common names between mice and humans, gene symbols of mice were matched with those of humans. Using these gene sets common to mice and humans, the calculation method described in connection with a mouse data set was applied. The whole-organ RNA-Seq data for human heart and kidney was acquired from "The Human Protein Atlas"(https://www.proteinatlas.org/).

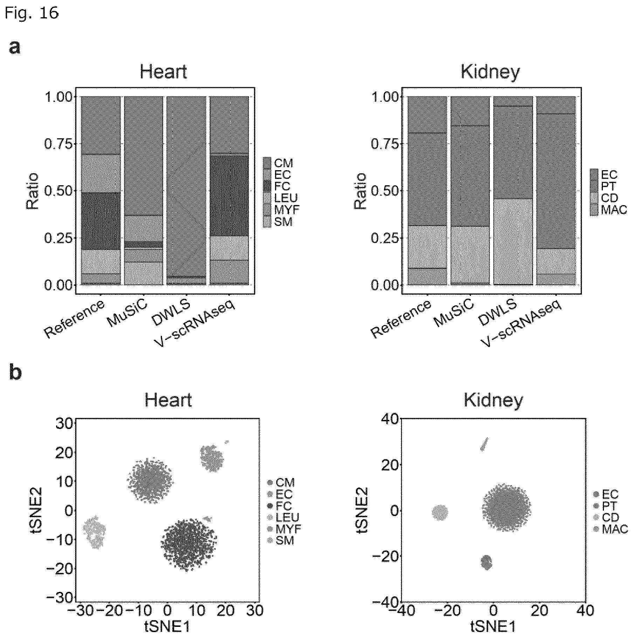

The results are shown in FIG. 8. It was shown that the composition ratios of cell types calculated for heart and kidney of humans are similar to the composition ratios of cell types in corresponding organs of normal mice (FIG. 16a). Further, the results of analysis of t-SNE of estimated scRNA-Seq data of heart and kidney of humans showed that classification based on the gene expression profiles of known cell types in each organ is possible (FIG. 16b). These results indicate the cross-species applicability of the cell type-specific weight coefficients and the V-scRNASeq framework.

<List of composition ratios of reference cell types>

In the following list, the items are sorted in the order of Organ:Cell type:Abbreviation:Reference. The ";" is intended to mean a delimiter of data for each cell type. The cell composition ratios are normalized such that the whole-organ is "1." Because representative cell types are shown here, the sum of the composition ratios of respective cell types in each organ is not necessarily equal to 1.

Aorta:Aorta-endothelial cell-NA:EC :0.40 ;
Aorta:Aorta-erythrocyte-NA:ERC:0.21 ;
Aorta:Aorta-fibroblast-NA:FC :0.22 ;
Aorta:Aorta-professional antigen presenting cell-NA:PAP:0.16 ;
Brain: Brain_Myeloid-macrophage-NA:MAC:0.00 ;
Brain:Brain_Myeloid-microglial cell-NA:MI :0.10 ;
Brain:Brain_Non-Myeloid-astrocyte-NA:AS :0.02 ;
Brain:Brain_Non-Myeloid-Bergmann glial cell-NA:BGC:0.00 ;
Brain:Brain_Non-Myeloid-brain pericyte-NA:BP :0.02 ;
Brain:Brain_Non-Myeloid-endothelial cell-NA:EC :0.06 ;
Brain:Brain_Non-Myeloid-neuron-excitatory neurons and some neuronal stem cells:NEUR2 :0.47 ;
Brain:Brain_Non-Myeloid-neuron-inhibitory neurons:NEUR1 :0.21 ;
Brain:Brain_Non-Myeloid-oligodendrocyte-NA:OLC:0.09 ;
Brain:Brain_Non-Myeloid-oligodendrocyte precursor cell-NA:OPC:0.02 ;
Fat:Fat-B cell-NA:B:0.10 ;
Fat:Fat-endothelial cell-NA:EC :0.16 ;
Fat:Fat-mesenchymal stem cell of adipose-mesenchymal progenitor:MSA:0.43 ;
Fat:Fat-myeloid cell-NA:MYE:0.20 ;
Fat:Fat-NA-NA:NA :0.01 ;
Fat:Fat-natural killer cell-NA:NK :0.01 ;
Fat:Fat-T cell-NA:T:0.08 ;
Heart:Heart-cardiac muscle cell-NA:CM :0.30 ;
Heart:Heart-endocardial cell-NA:ECC:0.02 ;
Heart:Heart-endothelial cell-NA:EC :0.20 ;

Heart:Heart-fibroblast-NA:FC :0.29 ;
Heart:Heart-leukocyte-NA:LEU:0.13 ;
Heart:Heart-myofibroblast cell-NA:MYF:0.05 ;
Heart:Heart-NA-conduction cells:CC :0.01 ;
Heart:Heart-smooth muscle cell-NA:SM :0.01 ;
Kidney:Kidney-endothelial cell-NA:EC :0.19 ;
Kidney:Kidney-epithelial cell of proximal tubule-NA:PT :0.48 ;
Kidney:Kidney-kidney collecting duct epithelial cell-NA:CD :0.22 ;
Kidney:Kidney-leukocyte-NA:LEU:0.02 ;
Kidney:Kidney-macrophage-NA:MAC:0.09 ;
Large Intestine:Large Intestine-Brush cell of epithelium proper of large intestine-Tuft cell:TUF:0.01 ;
Large Intestine:Large Intestine-enterocyte of epithelium of large intestine-Enterocyte (Distal):END :0.06 ;
Large Intestine:Large Intestine-enterocyte of epithelium of large intestine-Enterocyte (Proximal):EN-P :0.21 ;
Large Intestine:Large Intestine-enteroendocrine cell-Chromaffin Cell:CHR:0.01 ;
Large Intestine:Large Intestine-epithelial cell of large intestine-Lgr5- amplifying undifferentiated cell:EP1:0.16 ;
Large Intestine:Large Intestine-epithelial cell of large intestine-Lgr5- undifferentiated cell:EP2:0.10 ;
Large Intestine:Large Intestine-epithelial cell of large intestine-Lgr5+ amplifying undifferentiated cell (Distal):EP3-D:0.03 ;
Large Intestine:Large Intestine-epithelial cell of large intestine-Lgr5+ amplifying undifferentiated cell (Proximal):EP3-P:0.05 ;
Large Intestine:Large Intestine-epithelial cell of large intestine-Lgr5+ undifferentiated cell (Distal):EP4-D:0.08 ;
Large Intestine:Large Intestine-epithelial cell of large intestine-Lgr5+ undifferentiated cell (Proximal):EP4-P:0.12 ;
Large Intestine:Large Intestine-large intestine goblet cell-Goblet cell (Distal):GB1-D:0.09 ;
Large Intestine:Large Intestine-large intestine goblet cell-Goblet cell (Proximal):GB1-P:0.05 ;
Large Intestine:Large Intestine-large intestine goblet cell-Goblet cell, top of crypt (Distal):GB2-D:0.02 ;
Liver:Liver-B cell-NA:B:0.07 ;
Liver:Liver-endothelial cell of hepatic sinusoid-NA:EC :0.33 ;
Liver:Liver-hepatocyte-NA:HE :0.42 ;
Liver:Liver-Kupffer cell-NA:KUP:0.11 ;
Liver:Liver-natural killer cell-NK/NKT cells:NK2:0.07 ;
Lung:Lung-B cell-NA:B:0.02 ;
Lung:Lung-ciliated columnar cell of tracheobronchial tree-multiciliated cells:CCC:0.01 ;
Lung:Lung-classical monocyte-invading monocytes:CMN:0.07 ;
Lung:Lung-epithelial cell of lung-alveolar epithelial type 1 cells, alveolar epithelial type 2 cells, club cells, and basal cells:EP5:0.06 ;
Lung:Lung-leukocyte-mast cells and unknown immune cells:LEU2 :0.02 ;
Lung:Lung-lung endothelial cell-NA:EC :0.34 ;
Lung:Lung-monocyte-circulating monocytes:MN2:0.07 ;
Lung:Lung-myeloid cell-dendritic cells, alveolar macrophages, and interstital macrophages:MYE2 :0.01 ;
Lung:Lung-NA-lung neuroendocrine cells and unknown cells:NC :0.03 ;
Lung:Lung-natural killer cell-NA:NK :0.02 ;
Lung:Lung-stromal cell-NA:SC :0.33 ;
Lung:Lung-T cell-NA:T:0.03 ;
Marrow:Marrow-B cell-Cd3e+ Klrb1+ B cell:B2 :0.01 ;
Marrow:Marrow-basophil-NA:BAS:0.00 ;
Marrow:Marrow-common lymphoid progenitor-NA:CLP:0.04 ;
Marrow:Marrow-granulocyte-NA:GRA:0.16 ;
Marrow:Marrow-granulocyte monocyte progenitor cell-NA:GMP:0.02 ;
Marrow:Marrow-granulocytopoietic cell-NA:GC :0.05 ;
Marrow:Marrow-hematopoietic precursor cell-NA:HPC:0.08 ;
Marrow:Marrow-immature B cell-NA:IB :0.06 ;
Marrow:Marrow-immature natural killer cell-NA:INK:0.01 ;
Marrow:Marrow-immature NK T cell-NA:INKT :0.01 ;
Marrow:Marrow-immature T cell-NA:IT :0.02 ;
Marrow:Marrow-late pro-B cell-Dntt- late pro-B cell:LPB1 :0.04 ;
Marrow:Marrow-late pro-B cell-Dntt+ late pro-B cell:LPB2 :0.03 ;
Marrow:Marrow-macrophage-NA:MAC:0.03 ;
Marrow:Marrow-mature natural killer cell-NA:MNT:0.01 ;

Marrow:Marrow-megakaryocyte-erythroid progenitor cell-NA:EPC:0.01 ;
Marrow:Marrow-monocyte-NA:MN :0.04 ;
Marrow:Marrow-naive B cell-NA:NBC:0.12 ;
Marrow:Marrow-pre-natural killer cell-NA:PNK:0.00 ;
Marrow:Marrow-precursor B cell-pre-B cell (Philadelphia nomenclature):PB :0.11 ;
Marrow:Marrow-regulatory T cell-NA:RT :0.00 ;
Marrow:Marrow-Slamf1-negative multipotent progenitor cell-NA:MPCI :0.10 ;
Marrow: Marrow-Slamf1-positive multipotent progenitor cell-NA:MPC2 :0.04 ;
SkMuscle:B cell_Jchain high(Muscle):B3 :0.02 ;
SkMuscle:B cell_Vpreb3 high(Muscle):B4 :0.09 ;
SkMuscle:Dendritic cell(Muscle):DEN:0.01 ;
SkMuscle:Endothelial cell(Muscle):EC :0.02 ;
SkMuscle:Erythroblast_Car1 high(Muscle):ERB1 :0.03 ;
SkMuscle:Erythroblast_Car2 high(Muscle):ERB2 :0.16 ;
SkMuscle:Granulocyte monocyte progenitor cell(Muscle):GMP:0.08 ;
SkMuscle:Macrophage_Ms4a6c high(Muscle):MAC2 :0.13 ;
SkMuscle:Macrophage_Retnla high(Muscle):MAC3 :0.02 ;
SkMuscle:Muscle cell_Tnnc1 high(Muscle):MC1:0.01 ;
SkMuscle:Muscle cell_Tnnc2 high(Muscle):MC2:0.03 ;
SkMuscle:Muscle progenitor cell(Muscle):MPC:0.08 ;
SkMuscle:Neutrophil_Camp high(Muscle):NEUT1 :0.16 ;
SkMuscle:Neutrophil_Prg2 high(Muscle):NEUT2 :0.01 ;
SkMuscle:Neutrophil_Retnlg high(Muscle):NEUT3 :0.12 ;
SkMuscle:Stromal cell(Muscle):SC :0.02 ;
SkMuscle:T cell(Muscle):T:0.01 ;
Pancreas:Pancreas-endothelial cell-NA:EC :0.06 ;
Pancreas:Pancreas-leukocyte-NA:LEU:0.04 ;
Pancreas:Pancreas-pancreatic A cell-pancreatic A cell:A:0.24 ;
Pancreas:Pancreas-pancreatic acinar cell-acinar cell:ACI:0.10 ;
Pancreas:Pancreas-pancreatic D cell-pancreatic D cell:D:0.11 ;
Pancreas:Pancreas-pancreatic ductal cell-ductal cell:DUC:0.12 ;
Pancreas:Pancreas-pancreatic PP cell-pancreatic PP cell:PP :0.05 ;
Pancreas:Pancreas-pancreatic stellate cell-stellate cell:PSC:0.04 ;
Pancreas:Pancreas-type B pancreatic cell-beta cell:BC :0.22 ;
Skin:Skin-basal cell of epidermis-Basal IFE:BE:0.22 ;
Skin:Skin-epidermal cell-Intermediate IFE:EPI :0.12 ;
Skin:Skin-keratinocyte stem cell-Inner Bulge:KSC:0.26 ;
Skin:Skin-keratinocyte stem cell-Outer Bulge:KSC2:0.37 ;
Skin:Skin-leukocyte-NA:LEU:0.01 ;
Skin:Skin-stem cell of epidermis-Replicating Basal IFE:SCE:0.02 ;
Spleen:Spleen-B cell-NA:B:0.77 ;
Spleen:Spleen-macrophage-NA:MAC:0.03 ;
Spleen:Spleen-T cell-NA:T:0.20 ;
Thymus:Thymus-DN1 thymic pro-T cell-DNI thymocytes:TPT:0.01 ;
Thymus:Thymus-immature T cell-DN4-DP in transition Cd69 negative rapidly dividing thymocytes:IT3:0.15 ;
Thymus:Thymus-immature T cell-DN4-DP in transition Cd69 negative thymocytes:IT2:0.44 ;
Thymus:Thymus-immature T cell-DN4-DP in transition Cd69 positive thymocytes:IT4:0.37 ;
Thymus:Thymus-leukocyte-antigen presenting cell:LEU3:0.02

<List of composition ratios of reference cell types>

In the following list, the items are sorted in the order of Organ:Singnature.gene.set.number:Cell.type:mean:var:min:first_quantile:Median:third_quantile: max. The ";" is intended to mean a delimiter of data for each cell type.

Aorta: 100:EC :0.151788089:0.320824524:0.01:0.01:0.01:0.020643025:4.333799883;
Aorta:100:ERC:24.67386955:27569.49096:0.01:0.268057658:0.947630617:4.647035248:1361.8 54647;
Aorta:100:FC :1.120387302:7.507603394:0.01:0.014564004:0.061908957:0.516121627:12.418 86869;

Aorta:100:PAP:0.124841086:0.130858196:0.01:0.01:0.01211706:0.032624313:1.661400716;
Aorta:300:EC :0.335653916:0.888982181:0.01:0.01:0.01:0.10214728:5.784560681;
Aorta:300:ERC:20.7856725:15487.87723:0.01:0.132647672:0.943529224:3.121387266:1008.04 8707;
Aorta:300:FC :1.122992247:3.637888239:0.01:0.103255397:0.318573613:1.121704441:9.9386 71176;
Aorta:300:PAP:0.16699144:0.283733571:0.01:0.01:0.014927526:0.085629113:3.587288911;
Aorta: 1577:EC :0.328052831:2.980101375:0.01:0.01:0.01:0.01:12.65695793;
Aorta: 1577:ERC:0.861381942:10.22004111:0.01:0.01:0.01:0.189752138:24.51986069;
Aorta: 1577:FC :1.157779979:11.62919843:0.01:0.01:0.011244537:0.227616723:17.14421124;
Aorta: 1577:PAP:0.236296791:0.806728782:0.01:0.01:0.01:0.01:4.644598908;
Brain:100:AS :0.434856565:2.615451954:0.01:0.022455987:0.046046996:0.12257169:18.3800 1032;
Brain:100:BGC:1.072299836:4.247525505:0.052380374:0.193697173:0.441824763:0.8154235 38:9.813487019;
Brain:100:BP:1.286448516:7.416001166:0.01:0.149224444:0.442108329:1.349882731:20.5549 2602;
Brain: 100:EC :0.155829289:2.482545762:0.01:0.011392245:0.018610335:0.058316551:33.770 84044;
Brain: 100:MAC:0.048124031:0.004108694:0.01:0.013496606:0.023131426:0.063089991:0.377 400612;
Brain: 100:MI :0.012961869:2.3118E-05:0.01:0.010033301:0.011173433:0.013679577:0.064623845;
Brain:100:NEUR1:2.653825355:114.4922959:0.01:0.014365065:0.065192324:0.488780763:74. 1873715;
Brain: 100:NEUR2 :1.516349011:26.86180756:0.01:0.017777754:0.088547977:0.515962167:38. 7956663;
Brain:100:OLC:1.47033239:789.3444746:0.01:0.043020985:0.137535878:0.575570849:1014.92 7739;
Brain: 100:OPC: 1.384613588:14.07919044:0.01:0.012646413:0.040728358:0.702904737:25.502 41672;
Brain:300:AS:1.74038249:13.24520375:0.01:0.015261047:0.195577225:1.884738702:41.4142 6285;
Brain:300:BGC:1.341778577:3.944690072:0.045824361:0.224444365:0.549709956:1.4584669 49:8.815660456;
Brain:300:BP:1.749492174:12.04296246:0.01:0.014499893:0.199956059:1.774169825:17.3753 9007;
Brain:300:EC :0.205803506:0.364383999:0.01:0.01:0.010304075:0.054289482:7.530690763;
Brain:300:MAC:0.010347881:4.91595E-06:0.01:0.01:0.01:0.01:0.024372891;
Brain:300:MI :0.010016051:1.25293E-07:0.01:0.01:0.01:0.01:0.02439228;
Brain:300:NEUR1:0.091026428:0.378856142:0.01:0.01:0.01:0.01:5.354404503;
Brain:300:NEUR2 :1.881598235:117.9200715:0.01:0.01:0.01:0.010376092:122.6759504;
Brain:300:OLC:0.683957014:4.665791152:0.01:0.01:0.031833092:0.255883967:30.43686688;
Brain:300:OPC:0.371699481:4.009370499:0.01:0.01:0.01:0.022522107:22.58268291;
Brain:2000:AS :1.591406611:8.974491561:0.01:0.01:0.070425016:1.748463704:15.85464919;
Brain:2000:BGC:1.125268038:5.864155062:0.01:0.01:0.013973665:0.729619368:9.858942038;
Brain:2000:BP :0.010231754:6.39702E-06:0.01:0.01:0.01:0.01:0.03860368;
Brain:2000:EC :0.046223928:0.054173766:0.01:0.01:0.01:0.01:2.487765951;
Brain:2000:MAC:0.010208662:4.75753E-07:0.01:0.01:0.01:0.01:0.013557299;
Brain:2000:MI :0.01008294:2.25461E-06:0.01:0.01:0.01:0.01:0.062541367;
Brain:2000:NEUR1:0.044018483:0.043085508:0.01:0.01:0.01:0.01:1.347998842;
Brain:2000:NEUR2 :1.008760807:29.81632686:0.01:0.01:0.01:0.01:51.96076429;
Brain:2000:OLC:0.55101755:2.370980823:0.01:0.01:0.01:0.153605575:17.776641;
Brain:2000:OPC:0.014554191:0.001657313:0.01:0.01:0.01:0.01:0.498116609;
Fat:100:B:0.046754003:0.011173744:0.01:0.010216413:0.015136787:0.037396414:1.30210247 7;
Fat:100:EC :2.414604433:19.41475516:0.01:0.010548113:0.280908337:2.789098145:41.78589 451;
Fat: 100:MSA:0.320323969:1.112225282:0.01:0.01:0.01:0.04529986:10.49111527;
Fat: 100:MYE:0.071228559:0.045200484:0.01:0.01:0.014329697:0.043635857:3.146909307;
Fat:100:NA:2.710777335:28.70862922:0.01:0.01:0.285071051:3.167316468:27.15404948;
Fat:100:NK :0.318498521:0.146633229:0.013489934:0.060943058:0.201014136:0.368195143:1 .823855366;
Fat: 100:T:0.45552476:2.077988331:0.01:0.01:0.029935117:0.292900664:16.31685769;
Fat:300:B:0.085593432:0.063367131:0.01:0.01:0.01:0.010472349:1.660266129;
Fat:300:EC:1.951699824:32.94181255:0.01:0.01:0.01:0.449707234:46.60358358;
Fat:300:MSA:0.301040647:2.273597383:0.01:0.01:0.01:0.01:20.45002588;
Fat:300:MYE:0.145061513:0.432465824:0.01:0.01:0.01:0.011508338:8.06361426;
Fat:300:NA :0.097562096:0.200890568:0.01:0.01:0.01:0.01:2.807266835;
Fat:300:NK :0.01:8.01339E-29:0.01:0.01:0.01:0.01:0.01;
Fat:300:T:0.010988287:0.000224172:0.01:0.01:0.01:0.01:0.237562315;
Fat:2000:B:0.267000961:1.17913974:0.01:0.01:0.01:0.012349464:10.30491319;
Fat:2000:EC :1.52498714:15.89721155:0.01:0.01:0.01:0.367139836:29.44172018;
Fat:2000:MSA:0.352538559:3.267249585:0.01:0.01:0.01:0.01:26.45242703;
Fat:2000:MYE:0.154984661:0.568213675:0.01:0.01:0.01:0.010339534:12.65610984;
Fat:2000:NA :0.038760219:0.01129055:0.01:0.01:0.01:0.01:0.520072065;

Fat:2000:NK :0.011812948:8.05709E-05:0.01:0.01:0.01:0.01:0.062738677;
Fat:2000:T:0.050755542:0.090293712:0.01:0.01:0.01:0.01:4.357269635;
Heart: 100:CC :0.01:4.19568E-32:0.01:0.01:0.01:0.01:0.01;
Heart: 100:CM :2.823889258:54.45296231:0.01:0.01:0.077634074:0.644519204:37.63426786;
Heart: 100:EC :0.383886505:8.95708179:0.01:0.01:0.0145107:0.127136156:60.17501651;
Heart: 100:ECC:0.142329405:0.121205364:0.01:0.012507964:0.028868296:0.164640401:2.3448 71042;
Heart:100:FC:0.089502111:0.035613316:0.01:0.01:0.013279019:0.057356411:1.287831061;
Heart: 100:LEU:0.049459847:0.008453581:0.01:0.011171809:0.016984044:0.053998062:1.1560 60563;
Heart:100:MYF:0.197874897:0.106739298:0.01:0.01282829:0.041756576:0.227034214:1.6807 78788;
Heart:100:SM:1.348022055:1.282516297:0.297989562:0.449770835:0.871973921:1.85423725 3:4.078581375;
Heart:300:CC:0.01:2.41086E-32:0.01:0.01:0.01:0.01:0.01;
Heart:300:CM:1.706492592:22.04625552:0.01:0.01:0.014028892:0.385791139:22.6212958;
Heart:300:EC:0.308624254:0.996382718:0.01:0.01:0.01441405:0.228046496:14.13284018;
Heart:300:ECC:0.075306832:0.026880368:0.01:0.01:0.018232343:0.066596459:1.01598398;
Heart:300:FC:0.080447546:0.034630326:0.01:0.01:0.012546049:0.04781712:1.673015079;
Heart:300:LEU:0.024387404:0.006626406:0.01:0.01:0.011895671:0.021707587:1.457680288;
Heart:300:MYF:0.292706873:0.433907162:0.01:0.011944381:0.075827223:0.295975505:6.112 36449;
Heart:300:SM:0.123010953:0.023413091:0.01:0.01:0.056597312:0.185566209:0.509664676;
Heart:2000:CC:0.010313811:7.39796E-07:0.01:0.01:0.01:0.01:0.01312443;
Heart:2000:CM:3.112064232:77.80962808:0.01:0.01:0.01:0.390638194:51.1228028;
Heart:2000:EC:0.13368653:0.143577296:0.01:0.01:0.01:0.040965266:2.829282942;
Heart:2000:ECC:0.310990008:4.392293003:0.01:0.01:0.01:0.01:14.68197108;
Heart:2000:FC:0.053694616:0.018926396:0.01:0.01:0.01:0.02231088:1.648764347;
Heart:2000:LEU:0.039521164:0.287158212:0.01:0.01:0.01:0.01:9.759389068;
Heart:2000:MYF:0.017612063:0.002417966:0.01:0.01:0.01:0.01:0.422266391;
Heart:2000:SM:0.077480204:0.044825118:0.01:0.01:0.01:0.01:0.817152076;
Kidney:100:CD:0.861873721:5.325131329:0.01:0.01:0.027677075:0.429648536:15.29691009;
Kidney:100:EC :0.087685384:0.053268255:0.01:0.01:0.010467171:0.079612624:1.530825402;
Kidney:100:LEU:0.019678869:0.000210734:0.01:0.01:0.01:0.029438172:0.048074249;
Kidney:100:MAC:0.014345637:0.00027765:0.01:0.01:0.01:0.01:0.096159876;
Kidney:100:PT:1.066404822:6.559136387:0.01:0.01:0.042416515:0.656508148:14.31773311;
Kidney:300:CD:1.02990839:31.96503195:0.01:0.01:0.01:0.01:46.00176013;
Kidney:300:EC :0.148567471:0.401009826:0.01:0.01:0.01:0.01:3.505286789;
Kidney:300:LEU:0.01:3.0112E-29:0.01:0.01:0.01:0.01:0.01;
Kidney:300:MAC:0.010736962:1.52072E-05:0.01:0.01:0.01:0.01:0.030634931;
Kidney:300:PT:1.078855015:17.30970861:0.01:0.01:0.01:0.01:34.86889454;
Kidney:1461:CD:0.571606592:3.043433891:0.01:0.01:0.01:0.082063639:10.68411549;
Kidney:1461:EC:0.262442826:0.506765362:0.01:0.01:0.01:0.040553741:4.187360011;
Kidney:1461:LEU:0.448563929:1.140902526:0.01:0.01:0.01:0.025451239:0.131769898:3.07252654 5;
Kidney:1461:MAC:0.015812234:0.000494913:0.01:0.01:0.01:0.01:0.126188915;
Kidney:1461:PT:1.009683719:9.1880931:0.01:0.01:0.010200921:0.349203618:18.39498163;
Large Intestine: 100:CHR:0.012506713:9.45398E-05:0.01:0.01:0.01:0.01:0.059484444;
Large Intestine: 100:EN-D :0.02890134:0.011490302:0.01:0.01:0.01:0.01:0.914454533;
Large Intestine: 100:EN-P :0.245917773:0.409541469:0.01:0.01:0.01:0.099277919:5.442989504;
Large Intestine:100:EP1:0.010344637:3.46044E-05:0.01:0.01:0.01:0.01:0.113368671;
Large Intestine:100:EP2:0.108748193:1.93259855:0.01:0.01:0.01:0.01:19.76858819;
Large Intestine: 100:EP3-D:0.589057488:2.322023876:0.01:0.01:0.036088665:0.212778459:7.753674897;
Large Intestine: 100:EP3-P:1.359012809:13.83295307:0.01:0.01:0.038106769:0.79029694:30.18008581;
Large Intestine: 100:EP4-D:1.334742196:8.776057103:0.01:0.01:0.051754177:0.765595964:15.7202124;
Large Intestine: 100:EP4-P:1.936669446:21.3101594:0.01:0.01:0.048946915:0.996926491:29.43809417;
Large Intestine:100:GB1-D:0.567448195:1.714325103:0.01:0.01:0.040470534:0.529872099:10.85628586;
Large Intestine:100:GB1-P:0.206993092:0.398629305:0.01:0.01:0.01:0.035904714:4.782087384;
Large Intestine:100:GB2-D:0.020528901:0.001429011:0.01:0.01:0.01:0.01:0.171902125;
Large Intestine:100:TUF:0.01:4.6781E-30:0.01:0.01:0.01:0.01:0.01;
Large Intestine:300:CHR:0.01:8.55585E-28:0.01:0.01:0.01:0.01:0.01;
Large Intestine:300:EN-D :0.028139446:0.0207549:0.01:0.01:0.01:0.01:1.423039804;
Large Intestine:300:EN-P :0.225660499:0.725122737:0.01:0.01:0.01:0.01:9.328144914;
Large Intestine:300:EPI:0.124803964:1.666430783:0.01:0.01:0.01:0.01:21.98275274;

Large Intestine:300:EP2:0.136387448:1.286467877:0.01:0.01:0.01:0.01:12.06235413;
Large Intestine:300:EP3-D:0.100450079:0.080406616:0.01:0.01:0.01:0.031250293:1.703547206;
Large Intestine:300:EP3-P:2.013779797:104.0491511:0.01:0.01:0.01:0.010203473:93.19098724;
Large Intestine:300:EP4-D:0.618244402:4.172513336:0.01:0.01:0.01:0.069821296:14.62455624;
Large Intestine:300:EP4-P:2.216779474:81.90841528:0.01:0.01:0.01:0.089417422:72.73878781;
Large Intestine:300:GB1-D:0.625078047:4.444453549:0.01:0.01:0.01:0.143932698:20.8490123;
Large Intestine:300:GB1-P:0.136413664:0.845071847:0.01:0.01:0.01:0.01:9.557555803;
Large Intestine:300:GB2-D:0.584599971:1.070237117:0.01:0.01:0.089996554:0.651617884:4.481089012;
Large Intestine:300:TUF:0.01:3.04504E-29:0.01:0.01:0.01:0.01:0.01;
Large Intestine:2000:CHR:0.015394585:0.000704757:0.01:0.01:0.01:0.01:0.153127464;
Large Intestine:2000:EN-D :0.058661679:0.087600473:0.01:0.01:0.01:0.01:2.68276365;
Large Intestine:2000:EN-P :0.186182828:0.342939218:0.01:0.01:0.01:0.018775138:4.579226957;
Large Intestine:2000:EP1:0.242031163:1.52035394:0.01:0.01:0.01:0.01:13.54761958;
Large Intestine:2000:EP2:0.137616207:0.684872242:0.01:0.01:0.01:0.01:7.342157204;
Large Intestine:2000:EP3-D:0.248573596:1.254007981:0.01:0.01:0.01:0.043345374:8.244720325;
Large Intestine:2000:EP3-P:1.160239961:8.681215788:0.01:0.01:0.01:0.196549515:14.92215724;
Large Intestine:2000:EP4-D:1.024156717:12.94504348:0.01:0.01:0.01:0.153132844:28.16052677;
Large Intestine:2000:EP4-P:1.870495516:32.95764143:0.01:0.01:0.01:0.241904836:37.58578581;
Large Intestine:2000:GB1-D:0.703346962:4.343873838:0.01:0.01:0.017259145:0.246563149:15.28585118;
Large Intestine:2000:GB1-P:0.267045238:3.849090953:0.01:0.01:0.01:0.01:20.24231902;
Large Intestine:2000:GB2-D:0.602863068:0.645603881:0.01:0.02646894:0.232151648:0.877725989:3.034948802;
Large Intestine:2000:TUF:0.01:6.86261E-32:0.01:0.01:0.01:0.01:0.01;
Liver:100:B:0.213797267:0.325687856:0.01:0.01:0.01:0.056571357:2.613370426;
Liver: 100:EC:0.037007003:0.043483818:0.01:0.01:0.01:0.01:2.711247341;
Liver: 100:HE:1.577528039:35.04314183:0.01:0.01:0.01:0.183535291:43.73524356;
Liver: 100:KUP:0.509042737:6.621034858:0.01:0.01:0.01:0.012229472:18.82181524;
Liver: 100:NK2:0.539076723:10.69009305:0.01:0.01:0.01:0.01:20.43361731;
Liver:300:B:0.011314986:7.08967E-05:0.01:0.01:0.01:0.01:0.063914422;
Liver:300:EC:0.113993526:0.733401923:0.01:0.01:0.01:0.01:10.81729271;
Liver:300:HE:1.561492365:98.12025078:0.01:0.01:0.01:0.01:105.9140195;
Liver:300:KUP:0.260603997:2.328280291:0.01:0.01:0.01:0.01:11.82954434;
Liver:300:NK2:0.125984933:0.524647684:0.01:0.01:0.01:0.01:4.533412393;
Liver:2000:B:0.046524022:0.014063618:0.01:0.01:0.01:0.01:0.629735622;
Liver:2000:EC:0.155958403:0.528364338:0.01:0.01:0.01:0.01:8.591024824;
Liver:2000:HE:1.498511944:62.89746473:0.01:0.01:0.01:0.01:83.09827976;
Liver:2000:KUP:0.255600958:1.055424386:0.01:0.01:0.01:0.04115651:7.789532314;
Liver:2000:NK2:0.367333689:2.537527345:0.01:0.01:0.01:0.01:9.737383429;
Lung:100:B:0.01857141:0.001028567:0.01:0.01:0.01:0.01:0.129999746;
Lung:100:CCC:1.456821314:8.825647444:0.01:0.01:0.01:1.547008977:9.746616082;
Lung:100:CMN:0.028888155:0.013039084:0.01:0.01:0.01:0.010769856:0.835026957;
Lung:100:EC:0.592266493:3.130564936:0.01:0.01:0.01:0.14637682:17.3946568;
Lung: 100:EP5:2.184258795:31.36444499:0.01:0.142788:0.468145799:1.199237162:33.160377 81;
Lung:100:LEU2 :0.01:2.14937E-25:0.01:0.01:0.01:0.01:0.01;
Lung: 100:MN2:0.015465605:0.000828191:0.01:0.01:0.01:0.01:0.194112768;
Lung:100:MYE2 :0.01115847:5.79673E-06:0.01:0.01:0.01:0.010703344:0.016013027;
Lung:100:NC:5.849657994:83.74798893:0.01:0.01:0.391509729:8.663540696:29.4471241;
Lung:100:NK:0.01:6.00811E-27:0.01:0.01:0.01:0.01:0.01;
Lung:100:SC:0.596743132:2.135538046:0.01:0.01:0.025279551:0.325975557:10.78671528;
Lung: 100:T:0.01:5.256E-27:0.01:0.01:0.01:0.01:0.01;
Lung:300:B:0.044904601:0.017056636:0.01:0.01:0.01:0.01:0.498664407;
Lung:300:CCC:1.891546254:5.51406788:0.01:0.056520696:0.298394181:3.445486709:6.05646 2974;
Lung:300:CMN:0.01072913:1.36084E-05:0.01:0.01:0.01:0.01:0.032120838;
Lung:300:EC :0.716551065:10.93183795:0.01:0.01:0.01:0.025238049:41.15891979;
Lung:300:EP5:2.367899563:40.49001777:0.01:0.01:0.075846939:1.72436971:34.37901999;
Lung:300:LEU2:0.01:3.0477E-29:0.01:0.01:0.01:0.01:0.01;
Lung:300:MN2:0.011887933:9.99892E-05:0.01:0.01:0.01:0.01:0.080417127;
Lung:300:MYE2 :0.01:4.19293E-28:0.01:0.01:0.01:0.01:0.01;

Lung:300:NC :3.512526692:52.14415724:0.01:0.01:0.166310027:3.261063433:26.50611444;
Lung:300:NK :0.01:8.33236E-28:0.01:0.01:0.01:0.01:0.01;
Lung:300:SC :0.453653037:1.848672295:0.01:0.01:0.01:0.044123625:8.947055684;
Lung:300:T:0.011154861:3.06752E-05:0.01:0.01:0.01:0.01:0.036561792;
Lung:2000:B:0.257379098:0.134236035:0.01:0.011041603:0.089877832:0.287607349:1.09485 4368;
Lung:2000:CCC:3.063528155:12.0082984:0.01:0.061665312:1.300096759:5.627807116:9.6987 05171;
Lung:2000:CMN:0.019441605:0.000835036:0.01:0.01:0.01:0.011049543:0.185526719;
Lung:2000:EC :0.690009542:4.988718328:0.01:0.01:0.01:0.165162893:19.51617638;
Lung:2000:EP5:1.706230301:7.732375989:0.01:0.065375787:0.461915327:1.751011363:10.15 66163;
Lung:2000:LEU2 :0.010031388:1.37926E-08:0.01:0.01:0.01:0.01:0.010439427;
Lung:2000:MN2:0.010778167:1.48816E-05:0.01:0.01:0.01:0.01:0.033806873;
Lung:2000:MYE2 :0.154800639:0.063766588:0.01:0.014829632:0.056271633:0.122672891:0.6 60438295;
Lung:2000:NC :2.838071299:26.0408083:0.01:0.01:0.011684946:2.709452458:15.53664066;
Lung:2000:NK :0.011379011:1.8491E-05:0.01:0.01:0.01:0.01:0.026888432;
Lung:2000:SC :0.325228195:1.505962612:0.01:0.01:0.01:0.040614985:13.08108162;
Lung:2000:T:0.435842093:0.882885258:0.01:0.01:0.037300932:0.313265777:4.183441938;
Marrow: 100:B2 :0.185902275:0.434241737:0.01:0.01:0.01:0.01:2.712418872;
Marrow:100:BAS:0.014519176:0.000265498:0.01:0.01:0.01:0.01:0.068749284;
Marrow:100:CLP:2.420824383:24.86621029:0.01:0.01:0.214101778:2.430900549:26.01299376
Marrow:100:EPC:1.904845804:16.12612784:0.01:0.01:0.210311004:1.707937823:19.04839729
Marrow:100:GC:1.621135952:4.828924043:0.01:0.117158592:0.707247043:2.393001514:11.2 4602065;
Marrow:100:GMP:0.181084318:0.236147535:0.01:0.01:0.016960511:0.092667507:3.47472325 1;
Marrow:100:GRA:0.374090145:0.702833462:0.01:0.012786779:0.047776796:0.259564272:7.9 14217848;
Marrow:100:HPC:1.13030154:12.97866174:0.01:0.01:0.01:0.195455929:27.27162771;
Marrow: 100:IB :0.31131546:5.521495558:0.01:0.01:0.01:0.01:32.72827775;
Marrow: 100:INK:0.01:3.00023E-30:0.01:0.01:0.01:0.01:0.01;
Marrow: 100:INKT :0.047342165:0.025679816:0.01:0.01:0.01:0.01:0.709047616;
Marrow: 100:IT :0.053571303:0.039218934:0.01:0.01:0.01:0.01:1.336584538;
Marrow:100:LPB1:1.608961936:8.767079358:0.01:0.01:0.177612782:1.660130475:16.040301 05;
Marrow:100:LPB2 :2.376398871:16.3857241:0.01:0.01:0.239978477:2.936407898:16.6589582 3;
Marrow: 100:MAC:0.0259025:0.006520404:0.01:0.01:0.01:0.01:0.564218133;
Marrow: 100:MN :0.038996541:0.026703762:0.01:0.01:0.01091448:0.019318394:1.81463237;
Marrow: 100:MNT :0.056297223:0.082362769:0.01:0.01:0.01:0.01:1.825894631;
Marrow:100:MPC1:0.367024786:1.315133774:0.01:0.01:0.01:0.090052996:9.312120992;
Marrow:100:MPC2:0.175320535:0.418543065:0.01:0.01:0.01:0.023679329:5.238568571;
Marrow:100:NBC:0.095766964:0.126490199:0.01:0.01:0.01:0.036544526:5.940112975;
Marrow:100:PB:0.010675228:0.000122283:0.01:0.01:0.01:0.01:0.218063081;
Marrow:100:PNK:2.482467108:17.15464909:0.01:0.022599142:0.036680956:3.944061666:14.7 4284385;
Marrow: 100:RT :0.012112594:6.19298E-05:0.01:0.01:0.01:0.01:0.040539169;
Marrow:300:B2 :0.072463781:0.080445354:0.01:0.01:0.01:0.01:1.517781713;
Marrow:300:BAS:0.518609643:3.362888996:0.01:0.01:0.01:0.01:6.621925359;
Marrow:300:CLP:1.284579894:25.89444494:0.01:0.01:0.01:0.047828455:31.57063454;
Marrow:300:EPC:0.975661564:17.3377232:0.01:0.01:0.01:0.017342857:20.88881381;
Marrow:300:GC :0.95647576:6.392217988:0.01:0.01:0.021089981:0.538024117:20.6035697;
Marrow:300:GMP:1.437037515:20.63563736:0.01:0.01:0.01:0.149559748:24.18271454;
Marrow:300:GRA:0.626599281:4.597668957:0.01:0.01:0.01:0.147438321:23.3880025;
Marrow:300:HPC:1.304453698:29.11832644:0.01:0.01:0.01:0.031238635:46.69346883;
Marrow:300:IB :0.581646271:13.28705952:0.01:0.01:0.01:0.01:40.52106243;
Marrow:300:INK:0.010677684:7.80735E-06:0.01:0.01:0.01:0.01:0.021520634;
Marrow:300:INKT :0.011091919:2.26534E-05:0.01:0.01:0.01:0.01:0.030746458;
Marrow:300:IT:0.187626221:0.516269114:0.01:0.01:0.01:0.01:4.414912871;
Marrow:300:LPB1:2.327177218:39.45478944:0.01:0.01:0.01:0.669150502:47.664881;
Marrow:300:LPB2:3.027541169:44.75653921:0.01:0.01:0.028413594:1.591414395:32.703420 5;
Marrow:300:MAC:0.533265095:3.571316901:0.01:0.01:0.01:0.038816232:13.60439309;
Marrow:300:MN :0.113166634:1.302606568:0.01:0.01:0.01:0.01:14.07338651;
Marrow:300:MNT:0.187233959:0.61635283:0.01:0.01:0.01:0.01:4.786642481;
Marrow:300:MPC1:0.201187132:1.513216898:0.01:0.01:0.01:0.01:14.12861891;
Marrow:300:MPC2 :0.049832787:0.104629922:0.01:0.01:0.01:0.01:3.562672271;

Marrow:300:NBC:0.186056236:4.138595569:0.01:0.01:0.01:0.01:37.74765959;
Marrow:300:PB :0.011186099:0.00027564:0.01:0.01:0.01:0.01:0.317098022;
Marrow:300:PNK:2.032067255:22.65497581:0.01:0.01:0.063921568:1.501403207:18.24728518
Marrow:300:RT:0.01:1.13792E-28:0.01:0.01:0.01:0.01:0.01;
Marrow:2000:B2 :0.167172108:0.259600952:0.01:0.01:0.01:0.01:2.487743147;
Marrow:2000:BAS:2.130613564:8.466073636:0.01:0.056819654:0.825334925:3.54974934:9.29 9718758;
Marrow:2000:CLP:1.098629192:10.50141526:0.01:0.01:0.01:0.265548246:28.26777702;
Marrow:2000:EPC:0.420817747:2.493197877:0.01:0.01:0.01:0.093986726:10.33754185;
Marrow:2000:GC :0.969074716:3.773959377:0.01:0.01:0.069629699:1.107661712:13.4267423 1;
Marrow:2000:GMP:1.71162988:8.833804552:0.01:0.051021256:0.494409338:1.397984403:12. 66009452;
Marrow:2000:GRA:0.675219767:3.340806905:0.01:0.01:0.014277322:0.347261219:14.961732 19;
Marrow:2000:HPC:0.894207305:5.34592313:0.01:0.01:0.01:0.400270601:20.58178485;
Marrow:2000:IB :0.369499113:2.05090549:0.01:0.01:0.01:0.010907813:14.24513112;
Marrow:2000:INK:0.056216304:0.027923716:0.01:0.01:0.01:0.01:0.699996817;
Marrow:2000:INKT :0.111411572:0.167818618:0.01:0.01:0.01:0.01:1.797976351;
Marrow:2000:IT:0.11687255:0.091009648:0.01:0.01:0.01:0.01347243:1.739893257;
Marrow:2000:LPB1:1.893858092:16.46027996:0.01:0.01:0.050292558:1.496103716:27.37277 965;
Marrow:2000:LPB2 :3.302615802:36.83218789:0.01:0.01:0.210685719:3.784145256:27.26089 787;
Marrow:2000:MAC:0.423312503:0.893001499:0.01:0.01:0.01:0.220012709:4.183752057;
Marrow:2000:MN :0.263946748:2.930026775:0.01:0.01:0.01:0.015794453:16.7846469;
Marrow:2000:MNT:0.181549588:0.183314907:0.01:0.01:0.01:0.029673303:1.778545776;
Marrow:2000:MPC1:0.362290945:1.790559251:0.01:0.01:0.01:0.035890566:14.05147694;
Marrow:2000:MPC2 :0.147777286:0.464731193:0.01:0.01:0.01:0.01:6.22391615;
Marrow:2000:NBC:0.137362883:0.348696493:0.01:0.01:0.01:0.01:7.142698962;
Marrow:2000:PB :0.058905676:0.294446787:0.01:0.01:0.01:0.01:9.928819957;
Marrow:2000:PNK:1.833011369:8.638914818:0.01:0.011389807:0.226801309:2.007042567:9.8 5192995;
Marrow:2000:RT :0.010730026:7.99407E-06:0.01:0.01:0.01:0.01:0.02095039;    Pancreas:100:A:0.01:2.95436E-27:0.01:0.01:0.01:0.01:0.01;
Pancreas:100:ACI:11.89919455:1239.078518:0.01:0.027755043:1.992745424:10.39200207:299 .3981303;
Pancreas:100:BC:0.014382556:0.001847759:0.01:0.01:0.01:0.01:0.531578068;
Pancreas:100:D:0.01:3.72485E-28:0.01:0.01:0.01:0.01:0.01;
Pancreas:100:DUC:0.085962055:0.033775105:0.01:0.01:0.012791323:0.057646561:1.32110618 4;
Pancreas:100:EC :9.397344192:1396.05103:0.01:0.01:0.188111294:0.979741716:253.3114757;
Pancreas:100:LEU:0.961108391:10.44635577:0.01:0.014391258:0.041267131:0.085491737:15. 68452118;
Pancreas: 100:PP :0.025692694:0.002933414:0.01:0.01:0.01:0.011266917:0.308624983;
Pancreas:100:PSC:2.221217553:63.14401388:0.01:0.01:0.054436934:0.626533357:46.1628028 2;
Pancreas:300:A:0.015787667:0.006866904:0.01:0.01:0.01:0.01:1.19647179;
Pancreas:300:ACI:13.64002226:1753.848566:0.01:0.01:0.01:2.740798527:312.48915;
Pancreas:300:BC:0.023946869:0.035699395:0.01:0.01:0.01:0.01:2.607600067;
Pancreas:300:D:0.01:1.69592E-25:0.01:0.01:0.01:0.01:0.01;
Pancreas:300:DUC:0.14580081:0.480585724:0.01:0.01:0.01:0.01:6.811039251;
Pancreas:300:EC :1.476887113:58.74553799:0.01:0.01:0.01:0.01:53.07849045;
Pancreas:300:LEU:2.617639833:230.4585141:0.01:0.01:0.01:0.01:93.58914858;
Pancreas:300:PP:0.01:3.8384E-25:0.01:0.01:0.01:0.01:0.01;
Pancreas:300:PSC:0.158792338:0.445999812:0.01:0.01:0.01:0.01:4.047027631;
Pancreas:2000:A:0.013012481:0.001619796:0.01:0.01:0.01:0.01:0.584870878;
Pancreas:2000:ACI:10.86705529:601.7602352:0.01:0.01:0.815365852:8.845002453:155.79986 31;
Pancreas:2000:BC :0.012869098:0.000777185:0.01:0.01:0.01:0.01:0.381544425;
Pancreas:2000:D:0.010021663:1.6756E-08:0.01:0.01:0.01:0.01:0.01106179;
Pancreas:2000:DUC:0.206309149:0.698594703:0.01:0.01:0.01:0.020448076:6.846125984;
Pancreas:2000:EC :0.624484527:6.177609328:0.01:0.01:0.01:0.022861064:16.26364263;
Pancreas:2000:LEU:0.224386478:1.390589728:0.01:0.01:0.01:0.016633662:7.289777506;
Pancreas:2000:PP :0.01169833:5.85563E-05:0.01:0.01:0.01:0.01:0.049072198;
Pancreas:2000:PSC:0.22808577:0.454643165:0.01:0.01:0.01:0.024362231:2.982198696;
Skin:100:BE:0.088525095:0.433917818:0.01:0.01:0.01:0.01:9.105400656;
Skin:100:EPI:1.497255989:8.07664797:0.01:0.023857561:0.156802628:1.353358422:15.12733 217;
Skin: 100:KSC:0.025219498:0.016150493:0.01:0.01:0.01:0.01:1.945234359;
Skin:100:KSC2:0.317196037:3.681240333:0.01:0.01:0.01:0.03712216:40.56973381;

Skin:100:LEU:6.537198302:190.7648029:0.01:0.01:0.076820057:6.008499214:46.96736574;
Skin:100:SCE:0.098152807:0.13388837:0.01:0.01:0.01:0.036533711:2.313070393;
Skin:300:BE:0.108357985:1.13393988:0.01:0.01:0.01:0.01:18.55862333;
Skin:300:EPI:1.305163535:21.44147441:0.01:0.01:0.01:0.098746599:36.14180497;
Skin:300:KSC:0.019268223:0.015211094:0.01:0.01:0.01:0.01:2.485425355;
Skin:300:KSC2:0.419843734:9.493485969:0.01:0.01:0.01:0.01:63.04223322;
Skin:300:LEU:7.856353791:227.1964209:0.01:0.01:0.01:10.01127642:50.34195125;
Skin:300:SCE:0.041529126:0.025377722:0.01:0.01:0.01:0.01:1.015417564;
Skin:2000:BE:0.054235631:0.107612828:0.01:0.01:0.01:0.01:4.894140689;
Skin:2000:EPI:1.463389585:13.46542866:0.01:0.01:0.041508019:0.580429537:24.10158418;
Skin:2000:KSC:0.057082183:0.283160697:0.01:0.01:0.01:0.01:8.947880368;
Skin:2000:KSC2:0.358296776:2.180414803:0.01:0.01:0.01:0.01:17.56111049;
Skin:2000:LEU:3.062859377:42.92355399:0.01:0.01:0.039353867:2.372801805:22.81755758;
Skin:2000:SCE:0.418997541:4.3441421:0.01:0.01:0.01:0.01:13.10047803;
SkMuscle:100:B3 :0.576226174:6.536569269:0.01:0.01:0.01:0.010556533:12.0156782;
SkMuscle:100:B4 :0.126395942:0.228262628:0.01:0.01:0.01:0.01:3.576026664;
SkMuscle:100:DEN:0.01:7.18298E-31:0.01:0.01:0.01:0.01:0.01;
SkMuscle:100:EC:0.675764223:2.800705161:0.01:0.01:0.030371209:0.285263553:7.31022740 7;
SkMuscle:100:ERB1:0.01:4.75706E-24:0.01:0.01:0.01:0.01:0.01;
SkMuscle: 100:ERB2 :0.010000162:4.76075E-12:0.01:0.01:0.01:0.01:0.010029355;
SkMuscle:100:GMP:0.087430963:0.486566491:0.01:0.01:0.01:0.01:6.365643955;
SkMuscle:100:MAC2:0.029195195:0.03837845:0.01:0.01:0.01:0.01:2.308267274;
SkMuscle:100:MAC3:0.013829597:0.000133662:0.01:0.01:0.01:0.01:0.06064265;
SkMuscle:100:MC1:28.93245718:212.9191713:0.101008:28.7416311:35.43885636:36.5236684 :44.08999214;
SkMuscle:100:MC2:15.04398917:182.2259111:0.01:5.74097169:11.36666489:21.01674324:54. 58031214;
SkMuscle:100:MPC:0.02286293:0.004120317:0.01:0.01:0.01:0.01:0.523549078;
SkMuscle:100:NEUT1:0.010017958:4.95878E-08:0.01:0.01:0.01:0.01:0.012908385;
SkMuscle: 100:NEUT2 :0.011664165:9.56584E-06:0.01:0.01:0.01:0.011539886:0.017153774;
SkMuscle: 100:NEUT3 :0.018413406:0.009697599:0.01:0.01:0.01:0.01:1.162636555;
SkMuscle: 100:SC :0.087895601:0.009532824:0.01:0.01:0.05575928:0.105801958:0.345386647;
SkMuscle:100:T:0.109161343:0.059970569:0.01:0.01:0.01:0.028898199:0.752215042;
SkMuscle:300:B3 :0.010361993:2.88286E-06:0.01:0.01:0.01:0.01:0.017963856;
SkMuscle:300:B4 :0.029963223:0.03376483:0.01:0.01:0.01:0.01:1.858624152;
SkMuscle:300:DEN:0.16913301:0.253233149:0.01:0.01:0.01:0.01:1.601330102;
SkMuscle:300:EC :0.895315862:11.40577034:0.01:0.01:0.01:0.01:16.86925061;
SkMuscle:300:ERB1:0.01:9.0575E-24:0.01:0.01:0.01:0.01:0.01;
SkMuscle:300:ERB2:0.010088532:1.41868E-06:0.01:0.01:0.01:0.01:0.026024377;
SkMuscle:300:GMP:0.037725117:0.063800617:0.01:0.01:0.01:0.01:2.311184743;
SkMuscle:300:MAC2:0.014416578:0.002691851:0.01:0.01:0.01:0.01:0.619487802;
SkMuscle:300:MAC3:0.010123343:3.80338E-07:0.01:0.01:0.01:0.01:0.013083577;
SkMuscle:300:MC1:7.592685729:87.58666453:0.01:1.765507418:4.031314363:10.95550861:3 0.46760561;
SkMuscle:300:MC2:15.5601171:833.7239616:0.01:0.01:1.157277635:12.19090123:106.330855 3;
SkMuscle:300:MPC:0.030102624:0.035158047:0.01:0.01:0.01:0.01:1.75892828;
SkMuscle:300:NEUT1:0.01:5.49289E-23:0.01:0.01:0.01:0.01:0.01;
SkMuscle:300:NEUT2:0.032082425:0.003901068:0.01:0.01:0.01:0.01:0.186659401;
SkMuscle:300:NEUT3:0.01087057:0.000103831:0.01:0.01:0.01:0.01:0.129268097;
SkMuscle:300:SC :0.033081414:0.009127034:0.01:0.01:0.01:0.01:0.465886089;
SkMuscle:300:T:0.010433177:1.68878E-06:0.01:0.01:0.01:0.01:0.013898597;
SkMuscle:2000:B3 :2.27902329:55.61744578:0.01:0.01:0.01:0.01:29.79690193;
SkMuscle:2000:B4 :0.081475145:0.454831686:0.01:0.01:0.01:0.01:6.820171116;
SkMuscle:2000:DEN:0.023446865:0.001476213:0.01:0.01:0.01:0.01:0.132248841;
SkMuscle:2000:EC:1.148049772:26.0796183:0.01:0.01:0.01:0.01:26.51870209;
SkMuscle:2000:ERB1:0.01:2.62163E-29:0.01:0.01:0.01:0.01:0.01;
SkMuscle:2000:ERB2:0.012219673:0.000496529:0.01:0.01:0.01:0.01:0.28302578;
SkMuscle:2000:GMP:0.010542119:2.43931E-05:0.01:0.01:0.01:0.01:0.054995847;
SkMuscle:2000:MAC2:0.09516298:0.982113219:0.01:0.01:0.01:0.01:11.65243136;
SkMuscle:2000:MAC3:0.01:2.2775E-30:0.01:0.01:0.01:0.01:0.01;
SkMuscle:2000:MCI:7.164084475:71.86606507:0.01:0.787027995:4.186646381:11.04237596: 27.32437377;

SkMuscle:2000:MC2:14.32686526:568.351699:0.01:0.156707198:2.07884161:13.74566834:82. 41595513;
SkMuscle:2000:MPC:0.274615674:3.129079571:0.01:0.01:0.01:0.01:14.27447261;
SkMuscle:2000:NEUT1 :0.012227059:0.00053056:0.01:0.01:0.01:0.01:0.306938325;
SkMuscle:2000:NEUT2 :0.01:2.46619E-30:0.01:0.01:0.01:0.01:0.01;
SkMuscle:2000:NEUT3 :0.215387785:5.34538476:0.01:0.01:0.01:0.01:27.06598056;
SkMuscle:2000:SC :0.384236497:3.218447212:0.01:0.01:0.01:0.01:8.613897029;
SkMuscle:2000:T:0.01:4.29784E-30:0.01:0.01:0.01:0.01:0.01;
Spleen:100:B:0.789739043:13.27629372:0.009999999:0.01:0.014974386:0.175114572:76.0135 3696;
Spleen:100:MAC:0.033677171:0.000928003:0.01:0.01052609:0.019320469:0.044604965:0.119 540695;
Spleen: 100:T:0.889478679:2.626844949:0.01:0.028523727:0.229516685:1.134817519:11.2108 9027;
Spleen:300:B:0.6007074:5.718042953:0.009999997:0.01:0.01:0.014069245:24.35418936;
Spleen:300:MAC:0.110306986:0.104400051:0.01:0.01:0.01:0.017623009:1.695440132;
Spleen:300:T:1.193135349:9.943389873:0.01:0.01:0.01:0.306849232:26.93225583;
Spleen:2000:B:0.527834996:2.847863632:0.01:0.01:0.01:0.026519544:14.2891133;
Spleen:2000:MAC:0.028947532:0.008489124:0.01:0.01:0.01:0.01:0.526640415;
Spleen:2000:T:0.902514553:4.96652535:0.01:0.01:0.01:0.263963255:13.63243794;
Thymus: 100:IT2:0.892838408:8.146903544:0.01:0.01:0.01:0.187870672:27.72681703;
Thymus: 100:IT3:1.517353985:8.980022847:0.01:0.01:0.087409456:1.088970761:12.81068485;
Thymus:100:IT4:0.509593999:2.415552664:0.01:0.01:0.019557589:0.222231944:12.92113673;
Thymus: 100:LEU3:0.075703947:0.050678587:0.01:0.01:0.01:0.010156311:0.855605674;
Thymus: 100:TPT:0.603227667:0.873439087:0.01:0.02145553:0.26503743:0.596217908:2.4324 18181;
Thymus:300:IT2:0.569771808:9.930095561:0.01:0.01:0.01:0.01:31.00492796;
Thymus:300:IT3:1.539814872:17.40773119:0.01:0.01:0.01:0.178454647:21.665993;
Thymus:300:IT4:0.293142261:3.327556195:0.01:0.01:0.01:0.01:19.65626539;
Thymus:300:LEU3:0.100077789:0.113494376:0.01:0.01:0.01:0.01:1.270564448;
Thymus:300:TPT:0.01:5.05421E-28:0.01:0.01:0.01:0.01:0.01;
Thymus:2000:IT2:0.461222048:2.278633079:0.01:0.01:0.01:0.015257748:11.52396779;
Thymus:2000:IT3:1.632787561:11.73465067:0.01:0.01:0.032282811:1.271299967:19.54368881
Thymus:2000:IT4:0.249174221:1.215448065:0.01:0.01:0.01:0.01:7.965207557;
Thymus:2000:LEU3:1.037253207:2.514034235:0.01:0.04187717:0.257041468:0.893381107:4.9 91732332;
Thymus:2000:TPT:0.011241477:9.2476E-06:0.01:0.01:0.01:0.01:0.017448864;

Reference Signs List

10: correcting device
101: control part
20: analyzing device
201: control part

**Claims**

1. A method for correcting a count data set for single-cell RNA-Seq analysis, comprising: weighting a count data set for single-cell RNA-Seq analysis obtained from cells to be analyzed or predicted for the cells to be analyzed based on the total RNA content of each cell type corresponding to the cells to be analyzed.

2. The correction method according to Claim 1, wherein the weighting is performed based on the expression of a signature gene set that characterizes each cell type, and the signature gene set includes a predetermined number of genes.

3. A method for analyzing single-cell RNA-Seq, comprising: weighting a count data set for single-cell RNA-Seq analysis obtained from cells to be analyzed or predicted for the cells to be analyzed based on the total RNA content of each cell type corresponding to the cells to be analyzed, and
analyzing an RNA expression pattern in each cell type composing an organ to be analyzed containing the cells to be analyzed based on the weighted count data set for single-cell RNA-Seq analysis.

4.  A method for analyzing composition ratios of cell types composing an organ to be analyzed, comprising:

    weighting a count data set for single-cell RNA-Seq analysis obtained from cells to be analyzed or predicted for the cells to be analyzed based on the total RNA content of each cell type corresponding to the cells to be analyzed, and
    analyzing the composition ratios of cell types composing an organ to be analyzed containing the cells to be analyzed based on the weighted count data set for single-cell RNA-Seq analysis.

5.  A device for correcting a count data set for single-cell RNA-Seq analysis, comprising a control part,
    wherein the control part weights a count data set for single-cell RNA-Seq analysis acquired from cells to be analyzed based on the total RNA content of each cell type corresponding to the cells to be analyzed.

6.  A device for analyzing single-cell RNA-Seq, comprising a control part,

    wherein the control part weights a count data set for single-cell RNA-Seq analysis obtained from cells to be analyzed or predicted for the cells to be analyzed based on the total RNA content of each cell type corresponding to the cells to be analyzed, and
    analyzes an RNA expression pattern in each cell type composing an organ to be analyzed containing the cells to be analyzed based on the weighted count data set for single-cell RNA-Seq analysis.

7.  A device for analyzing composition ratios of cell types composing an organ to be analyzed, comprising a control part,

    wherein the control part weights a count data set for single-cell RNA-Seq analysis obtained from cells to be analyzed or predicted for the cells to be analyzed based on the total RNA content of each cell type corresponding to the cells to be analyzed, and
    analyzes the composition ratios of cell types composing an organ to be analyzed containing the cells to be analyzed based on the weighted count data set for single-cell RNA-Seq analysis.

8.  A program for correcting a count data set for single-cell RNA-Seq analysis, executable by a computer to cause the computer to execute processing including a step of weighting a count data set for single-cell RNA-Seq analysis obtained from cells to be analyzed or predicted for the cells to be analyzed based on the total RNA content of each cell type corresponding to the cells to be analyzed.

9.  A program for analyzing single-cell RNA-Seq, executable by a computer to cause the computer to execute processing including steps of weighting a count data set for single-cell RNA-Seq analysis obtained from cells to be analyzed or predicted for the cells to be analyzed based on the total RNA content of each cell type corresponding to the cells to be analyzed, and
    analyzing an RNA expression pattern in each cell type composing an organ to be analyzed containing the cells to be analyzed based on the weighted count data set for single-cell RNA-Seq analysis.

10. A program for analyzing composition ratios of cell types composing an organ to be analyzed, executable by a computer to cause the computer to execute processing including steps of weighting a count data set for single-cell RNA-Seq analysis obtained from cells to be analyzed or predicted for the cells to be analyzed based on the total RNA content of each cell type corresponding to the cells to be analyzed, and
    analyzing the composition ratios of cell types composing an organ to be analyzed containing the cells to be analyzed based on the weighted count data set for single-cell RNA-Seq analysis.

Fig. 1

Fig. 2

```
        ┌─────────────┐
        │    Start    │
        └──────┬──────┘
               ▼
┌───────────────────────────────────────┐
│       Select a signature gene set.     │   S1
└───────────────────┬───────────────────┘
                    ▼
┌───────────────────────────────────────┐
│ Acquire scRNA-Seq count data of a      │
│ signature gene set for each cell type  │   S2
│ in an organ to be analyzed.            │
└───────────────────┬───────────────────┘
                    ▼
┌───────────────────────────────────────┐
│ Acquire whole-organ RNA-Seq count data │
│ of the organ to be analyzed.           │   S3
└───────────────────┬───────────────────┘
                    ▼
┌───────────────────────────────────────┐
│       Calculate weight coefficients    │   S4
└───────────────────┬───────────────────┘
                    ▼
┌───────────────────────────────────────┐
│ Acquire and store the weighted count   │
│ data set for scRNA-Seq analysis.       │   S5
└───────────────────┬───────────────────┘
                    ▼
        ┌─────────────┐
        │     End     │
        └─────────────┘
```

Fig. 3

Fig. 4

```
                        ┌─────────────┐
                        │    Start    │
                        └──────┬──────┘
                               │
                               ▼
┌──────────────────────────────────────────────────────────┐
│              Acquire an algorithm.                         │  S11
└──────────────────────────────────────────────────────────┘
                               │
                               ▼
┌──────────────────────────────────────────────────────────┐
│  Acquire whole-organ RNA-Seq count data of an organ to be │  S12
│                    analyzed.                               │
└──────────────────────────────────────────────────────────┘
                               │
                               ▼
┌──────────────────────────────────────────────────────────┐
│  Apply the whole-organ RNA-Seq count data and the weighted│  S13
│  count data set for scRNA-Seq analysis to the algorithm.  │
└──────────────────────────────────────────────────────────┘
                               │
                               ▼
┌──────────────────────────────────────────────────────────┐
│       Store and output the estimated results.             │  S14
└──────────────────────────────────────────────────────────┘
                               │
                               ▼
                        ┌─────────────┐
                        │     End     │
                        └─────────────┘
```

Fig. 5

Fig. 6

Fig. 7

Fig. 8

Fig. 9

Fig. 10

Fig. 11

Fig. 12

Fig. 13

Fig. 14

Fig. 15

Fig. 16

**a**

**b**

**EP 4 101 933 A1**

| INTERNATIONAL SEARCH REPORT | International application No. |
|---|---|
| | PCT/JP2021/004470 |

**A. CLASSIFICATION OF SUBJECT MATTER**
C12Q 1/6809(2018.01)i; C12Q 1/6869(2018.01)i
FI: C12Q1/6809 Z; C12Q1/6869
According to International Patent Classification (IPC) or to both national classification and IPC

**B. FIELDS SEARCHED**

Minimum documentation searched (classification system followed by classification symbols)
C12Q1/6809; C12Q1/6869

| Documentation searched other than minimum documentation to the extent that such documents are included in the fields searched | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | KANG, K. et al., "CDSeq: A novel complete deconvolution method for dissecting heterogeneous samples using gene expression data", PLOS Computational Biology, 2019, vol. 15, no. 12, e1007510, abstract, section "Materials and methods", lines 1-9, page 5, lines 9-16, etc. | 1-10 |
| X | MONACO, G. et al., "RNA-Seq Signatures Normalized by mRNA Abundance Allow Absolute Deconvolution of Human Immune Cell Types", Cell Reports, 2019, vol. 26, pp. 1627-1640, summary, page 1628, section "Modules of Cell Type-Specific Genes", pp. 1361-1362, section "mRNA Abundance", e5, section "Normalization for mRNA abundance", etc. | 1-10 |
| A | TSOUCAS, D. et al., "Accurate estimation of cell-type composition from gene expression data", NATURE COMMUNICATIONS, 2019, vol. 10, 2975, abstract, etc. | 1-10 |

☐ Further documents are listed in the continuation of Box C.  ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 09 April 2021 (09.04.2021) | 20 April 2021 (20.04.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DENG, Q. ; RAMSKOLD, D. ; REINIUS, B. ; SANDBERG, R.** *Science,* 2014, vol. 343, 193-196
- **HAN, X. et al.** Mapping the Mouse Cell Atlas by Microwell-Seq. *Cell,* 2018, vol. 172, 1091-1107
- **REGEV, A. et al.** Science Forum: The Human Cell Atlas. *Elife,* 2017, vol. 6
- **SANDBERG, R.** *Nature methods,* 2014, vol. 11, 22-24
- **TABULA MURIS, C. et al.** *Nature,* 2018, vol. 562, 367-372
- **ABBAS, A. R. et al.** *PloS one,* 2009, vol. 4, e6098
- **AVILA COBOS, F. et al.** *Bioinformatics,* 2018, vol. 34, 1969-1979
- **GAUJOUX, R. ; SEOIGHE, C.** *Infect Genet Evol,* 2012, vol. 12, 913-921
- **GONG, T. et al.** *PloS one,* 2011, vol. 6, e27156
- **GONG, T. ; SZUSTAKOWSKI, J. D.** *Bioinformatics,* 2013, vol. 29, 1083-1085
- **LI, B. et al.** *Genome biology,* 2016, vol. 17, 174
- **NEWMAN, A. M. et al.** *Nature methods,* 2015, vol. 12, 453-457
- **REPSILBER, D. et al.** *BMC bioinformatics,* 2010, vol. 11, 27
- **SHEN-ORR, S. S. ; GAUJOUX, R.** *Curr Opin Immunol,* 2013, vol. 25, 571-578
- **WANG, N. et al.** *Bioinformatics,* 2015, vol. 31, 137-139
- **ZHONG, Y. et al.** *BMC bioinformatics,* 2013, vol. 14, 89
- **TSOUCAS, D. et al.** *Nat Commun,* 2019, vol. 10, 2975
- **WANG, X. et al.** *Nat Commun,* 2019, vol. 10, 380
- **KANG, K. et al.** *PLoS computational biology,* 2019, vol. 15, e1007510